# EUROPEAN PATENT SPECIFICATION

(11) **EP 3 405 044 B1**
(45) Date of publication and mention of the grant of the patent: **23.12.2020**
(21) Application number: 17740944.8
(22) Date of filing: 23.01.2017
(51) Int. Cl.: A23L 3/3571, A23B 4/22, A23B 7/155, A23K 10/16, A23K 30/00, A23L 3/3472, A23L 3/3562, A61L 2/16, C12N 1/20

(54) **FOOD BIOPRESERVATIVE COMPOSITION AND USES THEREOF**
BIOKONSERVIERUNGSZUSAMMENSETZUNG FÜR LEBENSMITTEL UND VERWENDUNGEN DAVON
COMPOSITION BIOCONSERVATRICE ALIMENTAIRE ET SES UTILISATIONS

(30) Priority: 22.01.2016 CO 1615040 0
(43) Date of publication of application: 28.11.2018
(73) Proprietor: Solutions Biologiques Intelligents-Biointelligenza Inc., Quebec, Quebec G1X 2M2 (CA)
(72) Inventor: PEREZ ACOSTA, Adriana Alejandra, Sherbrooke, Québec J1L 1A4 (CA)
(74) Representative: Grosset-Fournier, Chantal Catherine
(86) International application number: PCT/CA2017/050068
(87) International publication number: WO 2017/124197

(56) References cited:
- WO-A1-2006/097415
- WO-A1-2013/185344
- CA-A1- 2 833 937
- CA-A1- 2 915 979
- US-B2- 6 780 447
- WEINBERG Z G ET AL: "The effect of Lactobacillus buchneri and L. plantarum, applied at ensiling, on the ensiling fermentation and aerobic stability of wheat and sorghum silages", JOURNAL OF INDUSTRIAL MICROBIOLOGY AND BIOTECHNOLOGY, BASINGSTOKE, GB, vol. 23, 10 November 1999 (1999-11-10), pages 218-222, XP002218368, ISSN: 1367-5435, DOI: 10.1038/SJ.JIM.2900726

## Description

### CROSS REFERENCE TO RELATED APPLICATIONS

This application claims benefit, of Columbian Patent Application Serial No. 16 15040 0000 0000, filed on January 22, 2016.

### STATEMENT REGARDING FEDERALLY SPONSORED RESEARCH OR DEVELOPMENT

N.A.

### FIELD OF THE INVENTION

The present invention relates to compositions used for food preservation and safety and to sanitize inanimate surfaces and to uses thereof. More specifically, the present invention concerns biopreservative compositions comprising a specific combination of cooperating bacteria (consortium of lactic acid bacteria) and an activating agent (energy source) which can be used in fresh and cooked food products and on inanimate surfaces to inhibit the growth of pathogenic bacteria and promote food safety and preservation.

### BACKGROUND OF THE INVENTION

Food safety has been geared towards finding solutions that provide answers to the risk of contamination caused by various pathogenic microorganisms, to whom are exposed foods, in particular, processed foods. During processing and storage, it is possible that pathogenic microorganisms grow and affect the nutritional properties of the food, generating undesirable organoleptic changes and facilitating the production of toxins.

The constant search for adequate food safety has driven the development of increasingly effective mechanisms, aimed towards the mitigation of pathogens microorganisms such as *Salmonella spp, Staphylococcus aureus, Yersinia enterocolitica, Clostridium botulinum, Clostridium perfringens, Campylobacter spp, Escherichia coli enterohemorrhagic* (O157: H7), *Listeria monocytogenes, Aspergillus niger, Aspergillus flavus* and *Aspergillus Rhizopus,* which can contaminate the food during its processing, storage and distribution, causing diseases to consumers.

Processed foods are those that result from a transformation of the raw material, and that are mixed with other substances (e.g. binders, flavorings, preservatives) to provide them improved organoleptic properties. Among processed foods are included pâtés, sausages, mortadella, ham and the whole line of cold cuts, pickled vegetables and whole fruits in syrup.

There are conservation systems that are being currently used in the processed food industry in order to stop food contamination by pathogenic microorganisms and extend the shelf life of food. Among the various preservatives for such foods, are chemical preservatives (e.g. nitrites and nitrates) and natural preservatives (e.g. ascorbic acid and lactic acid). The first ones have a considerable carcinogen potential in humans and do not inhibit completely the growth of contaminating microorganisms, while the second ones generate organoleptic alterations in the product due to the high concentration in which they should be used.

Biopreservatives are substances biologically produced by microorganisms (starters crops) that have proven lethal to pathogens and can be used to preserve different types of food, including processed foods. Biopreservative intake is safe in humans and may even be beneficial for the proper functioning of the body, such as in the case of probiotics. The use of certain strains of microorganisms as starter cultures (starters), can help ensure the safety of biopreservatives and additionally confer and / or improve organoleptic and nutritional characteristics of food.

Various products containing one or more microorganisms with nutritional and biopreservation purposes have been developed in the food manufacturing field. US patent No. 6780447 describes a product comprising sorbic acid and at least one bacteriocin produced by *Lactobacillus delbrueckii var. bulgaricus* and *Streptococcus thermophilus* which can be used either individually or together with other additives in agriculture, more particularly in feed for breeding cattle.

US patent application publication US20140271994 discloses a composition for feeding pets meat with microbial cultures, comprising at least one microorganism selected from *Pediococcus acidilactici*, *Pediococcus pentosaceus, Lactococcus lactic, Lactococcus cremoris, Lactobacillus delbrueckii var. bulgaricus, Lactobacillus plantarum, Lactobacillus pentosus, Streptococcus thermophilus, Lactobacillus sakei* and *Lactobacillus curvatus.*

US patent application publication US20100272854 describes a composition and method for increasing the viability of *bifidobacteria* type microorganisms during storage of fermented fresh food products containing one or more strains of *Bifidobacterium* and acacia. This species is known for being a powerful probiotic, but its high cost limits its use as a preservative. WO 2006/097415 A1 discloses a mixture of lactic acid bacterial and / or bifidobacteria in the manufacture of sourdough. Weinberg et al., Journal of Industrial Microbiology and Biotechnology, 1999, 23, pages 218-222, discloses the effect of Lactobacillus buchneri and L.plantarum, applied at ensiling. WO 2013/185344 A1 discloses the production of lactic acid from fermentations using mixed bacterial cultures. CA 2 833 937 A1 discloses lactic bacteria and compositions containing them against bacterial colds.

While a variety of preservative compositions for processed as well as unprocessed (fresh) food exists, there remains a need to develop new preservative compositions that are more effective in inhibiting the growth of pathogenic microorganisms while maintaining the organoleptic properties and extending the shelf life of food products.

### SUMMARY OF THE INVENTION

The inventors have found that some strains of lactic acid bacteria (LAB) exhibit a synergistic behavior when combined in defined proportions and formulated together with a specific activating agent as a biopreservative composition. The biopreservative composition was shown to effectively inhibit the growth of pathogenic microorganisms for longer periods of time (days) and to maintain or even improve the organoleptic properties of food products.

Accordingly, the present invention relates to a biopreservative composition comprising a consortium of LAB together with an activating agent, and optionally a vehicle. The present invention is defined by the appended claims.

In a first aspect, the present invention provides a food biopreservative composition comprising (i) a consortium of lactic acid bacteria comprising (a) at least one homofermentative lactic acid bacterium and (b) at least one heterofermentative lactic acid bacterium; and (ii) an activating agent, said biopreservative composition being as in the appended claims.

In embodiments (i) the at least one homofermentative lactic acid bacterium produces proteases; and the at least one homofermentative lactic acid bacterium and the at least one heterofermentative lactic acid bacterium are in a ratio (1:1); (ii) the at least one homofermentative lactic acid bacterium produce proteases; and the at least one homofermentative lactic acid bacterium and the at least one heterofermentative lactic acid bacterium are in a ratio (2:1); (iii) the at least one homofermentative lactic acid bacterium produce proteases; and the at least one homofermentative lactic acid bacterium and the at least one heterofermentative lactic acid bacterium are in a ratio (1:2); or (iv) the at least one homofermentative lactic acid bacterium is a probiotic lactic acid bacterium; and the at least one homofermentative lactic acid bacterium and the at least one heterofermentative lactic acid bacterium are in a ratio (1:3)

In embodiments, the consortium of lactic acid bacteria further comprises at least one probiotic lactic acid bacterium (either homofermentative or heterofermentative). In embodiments, (i) the at least one homofermentative lactic acid bacterium comprises at least one homofermentative lactic acid bacterium that produces proteases; and the at least one homofermentative lactic acid bacterium that produces proteases, the at least one heterofermentative lactic acid bacterium and the at least one probiotic lactic acid bacterium are in a ratio (1:1:1); (ii) the at least one homofermentative lactic acid bacterium comprises at least one homofermentative lactic acid bacterium that produces proteases; and the at least one homofermentative lactic acid bacterium that produces proteases, the at least one heterofermentative lactic acid bacterium and at least one probiotic lactic acid bacterium are in a ratio (1: 2: 1); or (iii) the at least one homofermentative lactic acid bacterium comprises at least one homofermentative lactic acid bacterium that produces proteases; and the at least one homofermentative lactic acid bacterium that produces proteases, the at least one heterofermentative lactic acid bacterium and the at least one probiotic lactic acid bacterium are in a ratio (1:3:1). In embodiments, the at least one probiotic lactic acid bacterium is a homofermentative lactic acid bacterium.

In embodiments, the at least one probiotic lactic acid bacterium comprises or consists of *Bifidobacterium, Pediococcus, Lactobacillus reuteri, Lactobacillus johnsonii, Lactobacillus sake, Carnobacterium fish, Leuconostoc mesenteroides, Lactobacillus curvatus* or any combination of at least two thereof.

In embodiments, the at least one probiotic lactic acid bacterium comprises or consists of *Bifidobacterium, Lactobacillus reuteri, Lactobacillus johnsonii, Lactobacillus sake, Carnobacterium fish, Leuconostoc mesenteroides, Lactobacillus curvatus* or any combination of at least two thereof.

In particular embodiments, the at least one probiotic lactic acid bacteria does not comprise or consist of *Pediococcus.* In particular embodiments, the at least one probiotic lactic acid bacteria does not comprise or consist of *Lactobacillus reuteri.* In particular embodiments, the at least one probiotic lactic acid bacteria does not comprise or consist of *Leuconostoc mesenteroides.* In particular embodiments, the at least one probiotic lactic acid bacteria does not comprise or consist of *Lactobacillus johnsonii.* In particular embodiments, the at least one probiotic lactic acid bacteria does not comprise or consist of *Lactobacillus sake.* In particular embodiments, the at least one probiotic lactic acid bacteria does not comprise or consist of *Carnobacterium fish.* In particular embodiments, the at least one probiotic lactic acid bacteria does not comprise or consist of *Lactobacillus curvatus.*

The at least one homofermentative lactic acid bacterium consists of *Lactobacillus delbrueckii subsp. bulgaricus, Lactobacillus delbrueckii subsp. lactis, Lactobacillus delbrueckii subsp. leichmannii, Lactobacillus acidophilus, Lactobacillus amylophilus, Lactobacillus amylovorus, Lactobacillus animalis, Lactobacillus crispatus, Lactobacillus farciminis, Lactobacillus gasseri, Lactobacillus jensenii, Lactobacillus ruminis, Lactobacillus salivarius, Lactobacillus sharpeae, Lactobacillus vitulinus, Lactobacillus yamanashiensis* or any combination of at least two (e.g., two, three, at least three, etc.) thereof.

In particular embodiments, the at least one homofermentative lactic acid bacterium does not comprise or consist of *Lactobacillus acidophilus.* In particular embodiments, the at least one homofermentative lactic acid bacterium does not comprise or consist of *Lactobacillus amylophilus.* In particular embodiments, the at least one homofermentative lactic acid bacterium does not comprise of consist or *Lactobacillus amylovorus.* In particular embodiments, the at least one homofermentative lactic acid bacterium does not comprise or consist of *Lactobacillus animalis.* In particular embodiments, the at least one homofermentative lactic acid bacterium does not comprise or consist of *Lactobacillus crispatus.* In particular embodiments, the at least one homofermentative lactic acid bacterium does not comprise or consist of *Lactobacillus farciminis.* In particular embodiments, the at least one homofermentative lactic acid bacterium does not comprise or consist of *Lactobacillus gasseri.* In particular embodiments, the at least one homofermentative lactic acid bacterium does not comprise or consist of *Lactobacillus jensenii.* In particular embodiments, the at least one homofermentative lactic acid bacterium does not comprise or consist of *Lactobacillus ruminis.* In particular embodiments, the at least one homofermentative lactic acid bacterium does not comprise or consist of *Lactobacillus salivarius.* In particular embodiments, the at least one homofermentative lactic acid bacterium does not comprise or consist of *Lactobacillus sharpeae.* In particular embodiments, the at least one homofermentative lactic acid bacterium does not comprise or consist of *Lactobacillus vitulinus.* In particular embodiments, the at least one homofermentative lactic acid bacterium does not comprise or consist of *Lactobacillus yamanashiensis.*

The at least one heterofermentative lactic acid bacterium consists of *Streptococcus thermophilus, Leuconostoc, Pediococcus, Lactobacillus plantarum, Lactobacillus* rhamnosus, *Lactobacillus helveticus* or any combination of at least two (e.g., two, three, at least three, etc.) thereof.

In particular embodiments, the at least one heterofermentative bacterium does not comprise or consist of *Leuconostoc.* In particular embodiments, the at least one heterofermentative bacterium does not comprise or consist of *Pediococcus.* In particular embodiments, the at least one heterofermentative bacterium does not comprise or consist of *Lactobacillus plantarum.* In particular embodiments, the at least one heterofermentative bacterium does not comprise or consist of *Lactobacillus ramnosus.* In particular embodiments, the at least one heterofermentative bacterium does not comprise or consist of *Lactobacillus helveticus.*

In embodiments, the consortium of lactic acid bacteria comprises *Lactobacillus delbrueckii subsp. bulgaricus* and *Streptococcus thermophilus.*

In embodiments, the consortium of lactic acid bacteria further comprises *Bifidobacterium.*

In embodiments, the consortium of lactic acid bacteria consists of *Lactobacillus delbrueckii subsp. bulgaricus* and *Streptococcus thermophilus.* In particular embodiments, the consortium of lactic acid bacteria consists of *Lactobacillus delbrueckii subsp. bulgaricus* and *Streptococcus thermophilus* in a ratio of 1:1.

In embodiments, the concentration of each bacterial species of the consortium of lactic acid bacteria in the composition is between about 1x10⁴ and about 1x10⁸ CFU/ml. In embodiments, the total concentration of bacteria of the consortium of lactic acid bacteria in the composition is between 2x10⁴ and about 5x10⁸ CFU/ml. In embodiments, the total concentration of bacteria of the consortium of lactic acid bacteria is between 3.9x10⁶ and about 9.9 x10⁶ CFU/ml. In embodiments, the total concentration of bacteria in the consortium of lactic acid bacteria in the composition is between about 9.9 x10⁶ CFU/ml.

The activating agent in the composition consists of starch. In embodiments, the starch comprises or consists of potato starch, yam starch, cassava starch, rice starch, corn starch, wheat starch, plantain starch or any combination of at least two thereof. In embodiments, the starch has an amylose content higher or equal to 10% (w/w). In embodiments, the starch has an amylopectin content lower or equal to 90% (w/w). In embodiments, the starch has a ratio of amylose: amylopectin between about 17:83 (w/w) and about 24:76 (w/w).

In embodiments, the starch is cassava starch, yam starch, or a combination thereof. In embodiments, the starch is cassava starch and the concentration of the cassava starch in the composition is between about 0.05% (w/v) and about 1% (w/v). In embodiments, the concentration of the cassava starch in the composition is between about 0.1% (w/v) and about 0.3% (w/v).

In embodiments, the starch is yam starch and the concentration of the yam starch in the composition is between about 0.1% (w/v) and about 3% (w/v). In embodiments, the starch is yam starch and the concentration of the yam starch in the composition is between about 0.1% (w/v) and about 2% (w/v). In embodiments, the concentration of the yam starch in the composition is about 0.3% (w/v). In embodiments, the concentration of the yam starch in the composition is about 2% (w/v).

In embodiments, the activating agent comprises or consists of an extract (e.g., aqueous) from plantain peels and the concentration of the extract in the composition is between about 0.1% (w/v) and about 20% (w/v). In preferred embodiments, the activating agent is an extract from plantain peels and the concentration of the extract in the composition is between about 5% (w/v) and about 20% (w/v). In embodiments, the concentration of the extract from plantain peels in the composition is about 15% (w/v).

The activating agent consists of a carbohydrate having from 3 to 5 carbon atoms. In embodiments, the carbohydrate is Xylitol, D-Ribose, D-Arabinose, D-lyxose, D-Xylose, D-Threose, D-Erytrose, D-Ribulose, D-Xylulose, D-Erythrulose or any combination of at least two thereof. In embodiments, the concentration of carbohydrate in the composition is between about 0.1% (w/v) and about 2% (w/v). In embodiments, the carbohydrate is Xylitol and the concentration of Xylitol in the composition is about 1.5% (w/v).

The activating agent comprises a fructooligosaccharide (FOS). In embodiments, the FOS comprises or consists of fructan or inulin.

In embodiments, the composition of the present invention is provided in liquid form. In embodiments, the composition of the present invention is provided in lyophilized form.

In embodiments, the biopreservative composition of the present invention further comprises a vehicle (diluent or carrier (food grade)).

The present invention also provides a kit or commercial package for the preparation and use the above described food biopreservative composition. In embodiments, the kit comprises a consortium of lactic acid bacteria; and (ii) an activating agent as described above. In the kit, the consortium of lactic acid bacteria may be provided in lyophilized or liquid form. Similarly, the activating agent may also be provided in lyophilized or liquid form. The kit may further comprise instructions to use the kit for increasing the shelf-life of food, for treating an inanimate surface which comes into contact with food or for inhibiting the growth of a pathogenic microorganism in food or on inanimate surfaces which come into contact with food (e.g., during packaging or processing of food).

The present invention also concerns a method for increasing the shelf-life of food (e.g., fresh food; cooked food, processed food, dry food, canned food, etc.) comprising applying (contacting) the above-described food biopreservative composition to food or an inanimate surface which comes into contact with food.

The present invention further concerns a method for inhibiting the growth of a pathogenic microorganism in food comprising applying (contacting) the above-described food biopreservative composition to food or an inanimate surface (e.g., synthetic surface) which comes into contact with food.

The biopreservative composition of the present invention may optionally be activated prior to being put into contact with the food or inanimate surface to be treated (e.g., by incubating the biopreservative composition for e.g., 1-5h at 37°C), such that the adaptive phase of the bacterial consortium in the biopreservative composition begins prior to being contacted with the food or surface to be treated.

The present invention further concerns the use of the above-described biopreservative composition or kit for increasing the shelf-life of food or for treating an inanimate surface (e.g., a synthetic surface) which comes into contact with food.

The present invention also concerns the use of the above-described biopreservative composition or kit for inhibiting the growth of a pathogenic microorganism in food.

In embodiments, the pathogenic microorganism is *E. coli, Salmonella spp, Listeria spp, Staphylococcus aureus, Clostridium, Aspergillus niger Aspergillus flavus* or any combination or at least two thereof.

In embodiments, the above-noted food is fresh food. In embodiments, the above-noted food is processed and/or cooked food. In embodiments, the above-noted food is canned food. In embodiments, the above-noted food is raw meat (e.g., ground meat). In embodiments, the above-noted food is a fresh vegetable or fruit. In embodiments, the above-noted food is dry or wet food for pet. In embodiments, the above-noted food is treat for pet.

The present invention also provides a bacterial consortium and an activating agent for use in food biopreservative compositions.

### BRIEF DESCRIPTION OF THE DRAWINGS

In the appended drawings:

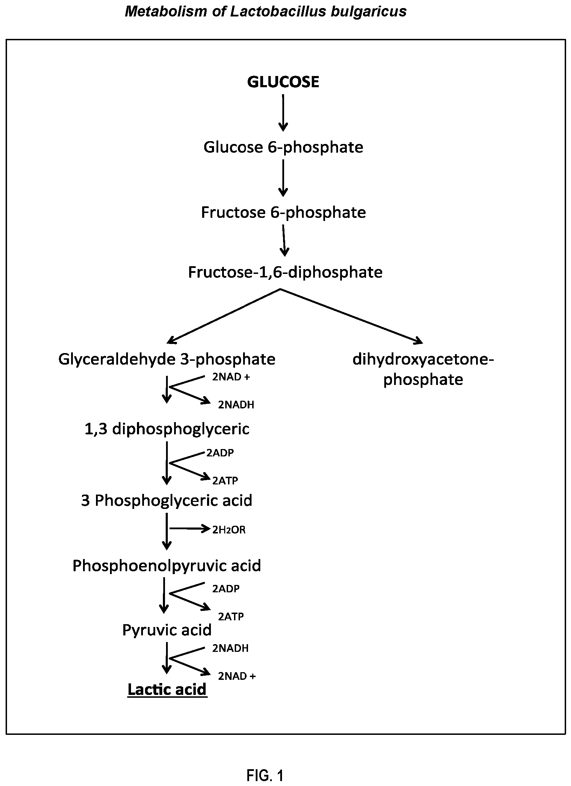

FIG. 1 shows a diagram of the metabolism of glucose of a homofermentative lactic acid bacteria (LAB) such as *Lactobacillus bulgaricus;*
FIG. 2 shows a diagram of the glucose metabolism of a heterofermentative LAB such as *Streptococcus thermophilus;*
FIG. 3 shows bacterial growth of a bacterial consortium of the present invention (1:1) of *Lactobacillus delbrueckii* subsp. *bulgaricus* and *Streptococcus thermophilus* on three meat substrates (pork (pig), chicken and sausage). Each curve represents the total number of bacteria *(Lactobacillus delbrueckii* subsp. *bulgaricus* and *Streptococcus thermophilus)* over time;
FIG. 4 shows the growth of a bacterial consortium of the present invention (1:1) of *Lactobacillus delbrueckii* subsp. *bulgaricus* and *Streptococcus thermophilus* in the presence of various carbon sources. (A) Cassava starch (0.1%) and xylitol (1.3%); (B) Peptone (0.2%) and glucose (0.01%);
FIG. 5 shows the chemical structure of cassava (tapioca) starch;
FIG. 6 shows the growth of a bacterial consortium of the present invention (1:1 of *Lactobacillus delbrueckii subsp. bulgaricus* and *Streptococcus thermophilus)* in the presence of various concentrations of yam *(Dioscorea rotundata)* starch;
FIG. 7 shows the growth of the bacterial consortium of the present invention (1:1) of *Lactobacillus delbrueckii subsp. bulgaricus* and *Streptococcus thermophilus* in the presence of a concentration of 15% of plantain peels extract (i.e., green bananas of the genus Musa whose fruit are generally used for cooking);
FIG. 8 shows the production process of a plantain peels (shells) extract for use as an activating agent in accordance with the present invention; and
FIG. 9 shows the inhibitory effect on the growth of *E. coli* O157: H7 in a sample of pork using the biopreservative composition of Example 2.

### DETAILED DESCRIPTION OF THE INVENTION

Lactic acid bacteria (LAB) are beneficial microorganisms composed of a large number of genera, including *Lactococcus, Lactobacillus, Enterococcus, Streptococcus, Leuconostoc, Pediococcus* and *Bifidobacterium* that are capable of inhibiting the growth of pathogenic or undesirable microorganisms in food, allowing extending its shelf life. A more detailed and complete description of the characteristics and properties of the LABs can be found in the review of Parra Huertas (1), which is incorporated by reference in its entirety.

LABs are widely used as starter cultures (starters) in the dairy industry as well as on meats, vegetables and grains. The initiators or starters cultures can be defined as a species or combination of microbial species that, once added to a food, initiate a set of transformations in its basic components (carbohydrate-protein-lipid), which translate into changes in the texture, color and flavor of food. Similarly, the starter cultures are capable of generating substances such as lactic acid, citric acid and CO₂, which favor the preservation of food.

The present invention relates to a biopreservative composition for food comprising a consortium of lactic acid bacteria, at least one activating agent and, optionally a vehicle. The biopreservative composition according to the invention may be liquid, semisolid or solid, and each of its components may be incorporated and/or formulated either individually or combined as a mixture.

In an aspect, the present invention provides a bacterial consortium of lactic acid bacteria comprising:
(i) at least one homofermentative lactic acid bacteria that produces proteases and at least one heterofermentative lactic acid bacteria, in a ratio (1:1);
(ii) at least one homofermentative lactic acid bacteria that produce proteases and at least one heterofermentative lactic acid bacteria, in a ratio (2:1);
(iii) at least one homofermentative lactic acid bacteria that produce proteases and at least one heterofermentative lactic acid bacteria, in a ratio (1:2).
(iv) at least one homofermentative probiotic lactic acid bacteria and at least one heterofermentative lactic acid bacteria, in a ratio (1: 3);
(v) at least one homofermentative lactic acid bacteria that produces proteases, at least one heterofermentative lactic acid bacteria and at least one probiotic lactic acid bacteria (either homofermentative or heterofermentative, preferably homofermentative), in a ratio (1:1: 1);
(vi) at least one homofermentative lactic acid bacteria that produces proteases, at least one heterofermentative lactic acid bacteria and at least one probiotic lactic acid bacteria (either homofermentative or heterofermentative, preferably homofermentative), in a ratio (1: 2: 1);
(vii) at least one homofermentative lactic acid bacteria that produces proteases, at least one heterofermentative lactic acid bacteria and at least one probiotic lactic acid bacteria (either homofermentative or heterofermentative, preferably homofermentative), in a ratio (1: 3: 1).

**Table 1: Non-limiting examples of homofermentative, heterofermentative and probiotic bacteria that may be included in the bacterial consortium of the present invention (see Samaniego Fernandez, Sosa del Castillo. Lactobacillis spp: importantes promotores de actividad probiótica, antimicrobiana y bioconservadora. Centro de estudios biotecnológicos. Facultad de Agronomia. Universidad de Matanzas "Camilo Cienfuegos". Matanzas, Cuba, which is incorporated herein by reference in its entirety).**

| **Homofermentative lactic acid bacteria that produces protease** | **Heterofermentative lactic acid bacteria** | **Probiotic lactic acid bacteria** |
|---|---|---|
| *Lactobacillus delbrueckii subsp. bulgaricus* | *Streptococcus thermophilus* | *Bifidobacterium* |
| *Lactobacillus delbrueckii subsp. lactis* | *Leuconostoc* | *Pediococcus* |
| *Lactobacillus delbrueckii subsp. leichmannii* | *Pediococcus* | *Lactobacillus reuteri* |
| *Lactobacillus acidophilus* | *Lactobacillus plantarum* | *Lactobacillus johnsonii* |
| *Lactobacillus amylophilus* | *Lactobacillus ramnosus* | *Lactobacillus sake* |
| *Lactobacillus amylovorus* | *Lactobacillus helveticus* | *Carnobacterium fish* |
| *Lactobacillus animalis* | | *Leuconostoc mesenteroides* |
| *Lactobacillus crispatus* | | *Lactobacillus curvatus* |
| *Lactobacillus farciminis* | | |
| *Lactobacillus gasseri* | | |
| *Lactobacillus jensenii* | | |
| *Lactobacillus ruminis* | | |
| *Lactobacillus salivarius* | | |
| *Lactobacillus sharpeae* | | |
| *Lactobacillus vitulinus* | | |
| *Lactobacillus yamanashiensis* | | |

It was found that a certain group of homofermentative lactic acid bacteria are not compatible with heterofermentative bacteria (e.g., *Streptococcus thermophilus*) used in the bacterial consortium of the present invention. The absence of cooperation and synergistic effect observed with these bacteria is thought to be due to the fact that they almost exclusively ferment hexoses (e.g., monosaccharides such as glucose, dextrose, mannose, allose, galactose, talose etc.). Therefore, in the presence of complex substrates (e.g., starch), amino acids required for the growth of *Streptococcus* are not produced at all or in insufficient amounts and cannot sustain the growth and activity of the heterofermentative bacteria (e.g., S. *thermophilus*) in the bacterial consortium. One important aspect of the present invention thus resides in the relationship that exists between the bacterial species in the bacterial consortium and its adaptation to the carbon source, enabling it to inhibit pathogenic microorganisms effectively.

**Table 2: Non-limiting examples of incompatible homofermentative lactic acid bacteria (see Samaniego Fernandez, Sosa del Castillo. Lactobacillis spp: importantes promotores de actividad probiótica, antimicrobiana y bioconservadora. Centro de estudios biotecnológicos. Facultad de Agronomia. Universidad de Matanzas "Camilo Cienfuegos". Matanzas, Cuba)**

| **Incompatible Lactic acid bacteria** |
|---|
| *Lactobacillus agilis* |
| *Lactobacillus alimentarius* |
| *L. casei subsp. casei* |
| *Lactobacillus homohiochii* |
| *Lactobacillus maltaromicus* |
| *Lactobacillus murinus* |

Furthermore, Applicants have found that *Bifidobacterium* and *Streptococcus thermophilus* cannot cooperate and need the presence of homofermentative lactic acid bacteria in order to be effective in a biopreservative composition. *Bifidobacterium* and *Streptococcus thermophilus* cannot be used alone as a bacterial consortium in accordance with the present invention.

In embodiments, the present invention provides a bacterial consortium comprising of at least two LAB selected from the group consisting of *Lactobacillus bulgaricus, Bifidobacterium* and *Streptococcus thermophilus* in a ratio 1:1, 1: 2, 1: 3, 1: 4, 1:1: 1, 1: 2: 1, 1: 3: 1 or 1: 2: 3 and in a concentration between 1x10⁴ and 1x10⁸ CFU/mL (e.g., 1x10⁴, 5x10⁴, 1x10⁵, 5x10⁵, 1x10⁶, 5x10⁶, 1x10⁷, 5x10⁷ and 1x10⁸ CFU/mL) for each microorganism. In a preferred embodiment, the consortium is made up of LAB *Lactobacillus bulgaricus* and *Streptococcus thermophilus* in ratio 1:1 and at a concentration of about 9,9x10⁶ CFU / mL of each bacterial species.

Strains of lactic acid bacteria of the biopreservative composition of the present invention can be obtained from commercially available cultures (*e.g.,* Clerici-Sacco, *Streptococcus t* 510, thermophilic cultures, SAP 230 A) or isolated from dairy products such as cheese or yogurt. For easy handling, strains can be stored by conventional lyophilization procedures.

The biopreservative of the present invention further comprises an activating agent which provides the bacterial consortium of the present invention, the energy necessary to activate its enzyme complex, start its growth in a controlled and cooperative manner (regulating the time in each of the phases) and to maintain its metabolic activity.

The activating agent according to the invention can be any compound that can serve as a source of energy for the bacterial consortium, including starch, glucose, dextrose, xylitol, peptone, whey and mixtures thereof. However, Applicant has found that certain energy sources work surprisingly well with the bacterial consortium of the present invention. Particularly useful energy (carbon) sources that may be used include starch (e.g., yam starch and cassava starch), xylitol (and other 3-5 carbons carbohydrates) and an extract of plantain peels. The use of such energy sources as activating agents in the biopreservative enables optimal bacterial growth and metabolic activity. The biopreservative was shown to start working rapidly and to maintain optimal activity over longer periods of time using these energy sources as activating agents.

Applicant has found that for the preparation of a biopreservative composition, the types of starch that may be used has an amylose content higher than or equal to 10% (w/w) or an amylopectin content lower than or equal to 90% (w/w). In embodiments, the ratio of amylose: amylopectin in the starch is at least 15:85 but does not exceed a ratio of 30:70 (w/w). Preferably, the starch has an amylose content higher than or equal to 17% (w/w) and an amylopectin content lower than or equal to 83% (w/w). In more preferred embodiments, the ratio of amylose: amylopectin in the starch is at least 17:83 but does not exceed a ratio of 24:76.

The use of starch appears to prolong the adaptation and exponential growth phases, thereby extending the period during which the biopreservative remains active against pathogenic bacteria. This is thought to be due to the more complex nature of starch, making more difficult to digest by the bacteria in in the consortium. The energy source is therefore digested more slowly and steadily.

**Table 3: Non-limiting examples of starch sources that may be used as an activating agent for the bacterial consortium of the present invention.**

| **Starch type** | **Amylose content (%)** | **Amylopectin content (%)** |
|---|---|---|
| Potato starch | 20 | 80 |
| Yam starch | 19 | 80 |
| Plantain starch | 24 | 76 |
| Wheat starch | 25 | 75 |
| Corn starch | 25 | 75 |
| Rice starch | 19 | 81 |
| Cassava Starch | 17 | 83 |

Furthermore, certain carbohydrates having in their structure between 3 to 5 carbons, such as FOS have also been shown to constitute a good source of energy for the bacterial consortium and may be included in the biopreservative of the present invention. Without being bound to any particular theory, it is though that in the presence of energy sources comprising a low carbon content (i.e., carbohydrates comprising 5 carbon atoms or less), consumption of the energy source remains relatively slow, possibly because bacteria in the consortium sense that the energy source is scarce.

**Table 4: Non-limiting examples of carbohydrates and FOS that may be used as an activating agent for the bacterial consortium of the present invention.**

| **Carbohydrate** | **Number carbons** |
|---|---|
| D-Ribose | 5 |
| D-Arabinose | 5 |
| D-Lyxose | 5 |
| D-Xylose | 5 |
| D-Threose | 4 |
| D-Erythrose | 4 |
| D-Ribulose | 5 |
| D-Xylulose | 5 |
| D-Erythrulose | 4 |

| **Fructooligosaccharides** | |
|---|---|
| fructans | 5 |
| inulin | 5 |

FOS are compounds whose structure is formed by repeating disaccharide units such as sucrose, inulobiose and levanobiose. Inulin (which belongs to the class of FOS) is a very heterogeneous polydispersed mixture of carbohydrates and polymers whose structure consists essentially of units of chain-terminating glucosyl moieties and a repetitive fructosyl moiety, which are linked by β(2,1) bonds with a polymerization degree ranging from 3 to 70 monomers.

The activating agent is present in the biopreservative of the invention either in solid form, in solution or dispersion in concentrations between 0.001% and 20.0% (w/w), more preferably between 0.05% and 15.0% (e.g., between 0.1% and 15 (w/w); between 0.2% and 10% (w/w); between 0.05% and 5% (w/w); between 0.05% and 2.5% (w/w), etc.).

In embodiments of the invention, the activating agent of the biopreservative is a dispersion of starch (e.g., potato, yam, wheat, corn, rice, plantain, cassava, etc.) in water at a concentration of between about 0.1% (w/v) and about 2% (w/v). In embodiments of the invention, the activating agent of the biopreservative is a dispersion of starch in water at a concentration of between about 0.1% (w/v) and about 0.5% (w/v). In embodiments of the invention, the activating agent of the biopreservative is a dispersion of starch in water at a concentration of between about 0.1% (w/v) and about 0.3% (w/v). In embodiments of the invention, the activating agent of the biopreservative is a dispersion of starch in water at a concentration of between about 0.1% (w/v) and about 0.4% (w/v). In embodiments of the invention, the activating agent of the biopreservative is a dispersion of starch in water at a concentration of between about 0.05% (w/v) and about 1% (w/v). In embodiments of the invention, the activating agent of the biopreservative is a dispersion of starch in water at a concentration of between about 0.05% (w/v) and about 0.5% (w/v). In embodiments of the invention, the activating agent of the biopreservative is a dispersion of starch in water at a concentration of between about 0.05% (w/v) and about 0.3% (w/v). In embodiments of the invention, the activating agent of the biopreservative is a dispersion of starch in water at a concentration of between about 0.05% (w/v) and about 0.4% (w/v). In embodiments of the invention, the activating agent of the biopreservative is a dispersion of starch in water at a concentration of 1.0% (w/v). In embodiments of the invention, the activating agent of the biopreservative is a dispersion of starch in water at a concentration of 0.1% (w/v). In embodiments of the invention, the activating agent of the biopreservative is a dispersion of starch in water at a concentration of 0.3% (w/v).

In embodiments, the starch comprises potato starch, cassava starch, rice starch, wheat starch, corn starch, yam starch and/or plantain starch.

In embodiments of the invention, the activating agent of the biopreservative is a dispersion of one or more (at least one) 3-5 carbons carbohydrate (e.g., xylitol, or a carbohydrate listed in Table 4 above) in water at a concentration of between about 0.05% (w/v) and about 5% (w/v). In embodiments of the invention, the activating agent of the biopreservative is a dispersion of one or more 3-5 carbons carbohydrate in water at a concentration of between about 0.1% (w/v) and about 5% (w/v). In embodiments of the invention, the activating agent of the biopreservative is a dispersion of one or more 3-5 carbons carbohydrate in water at a concentration of between about 0.1% (w/v) and about 3% (w/v). In embodiments of the invention, the activating agent of the biopreservative is a dispersion of one or more 3-5 carbons carbohydrate in water at a concentration of between about 0.1% (w/v) and about 2% (w/v). In embodiments of the invention, the activating agent of the biopreservative is a dispersion of one or more 3-5 carbons carbohydrate in water at a concentration of between about 0.1% (w/v) and about 1.5% (w/v). In embodiments of the invention, the activating agent of the biopreservative is a dispersion of one or more 3-5 carbons carbohydrate in water at a concentration of between about 0.05% (w/v) and about 5% (w/v). In embodiments of the invention, the activating agent of the biopreservative is a dispersion of one or more 3-5 carbons carbohydrate in water at a concentration of between about 0.05% (w/v) and about 3% (w/v). In embodiments of the invention, the activating agent of the biopreservative is a dispersion of one or more 3-5 carbons carbohydrate in water at a concentration of between about 0.05% (w/v) and about 2% (w/v). In embodiments of the invention, the activating agent of the biopreservative is a dispersion of one or more 3-5 carbons carbohydrate in water at a concentration of between about 0.05% (w/v) and about 1.5% (w/v).

In embodiments of the invention, the activating agent of the biopreservative is a dispersion of xylitol in water at a concentration of 1.5% (w/v). In embodiments, the activating agent of the biopreservative is a dispersion of cassava starch in water at a concentration of 0.1% (w/v). In embodiments, the activating agent of the biopreservative is a dispersion of plantain peels in water at a concentration of 15% (w/v). In embodiments, the activating agent of the biopreservative is a dispersion of yam starch in water at a concentration between 0.1% and 2% (w/v). In embodiments, the activating agent of the biopreservative is a dispersion of yam starch in water at a concentration of 2% (w/v). In embodiments, the activating agent of the biopreservative is a dispersion of yam starch in water at a concentration of 0.3% (w/v).

In embodiments, the activating agent of the biopreservative is a dispersion of a combination of anyone of (i) starch (e.g., cassava, yam potato, rice, plantain peels, etc.); (ii) carbohydrates having in their structure between 3 to 5 carbons; and (iii) a fructooligosaccharide (FOS) (e.g., fructans, inulin).

Additionally, the food biopreservative composition of the present invention may include an acceptable vehicle, consisting of different excipients with specific functions to shape or provide proper characteristics (e.g. forming solutions or dispersions, highlight organoleptic properties, increase its stability) and ensure that the bacterial consortium and the activating agent do not lose their ability to perform their function.

Among the excipients are included solvents, viscosifier agents, flavorings agents, preservatives, pH regulators, dyes, and in general all those commonly used and known by an expert in the art. The concentration of the vehicle in the biopreservative composition of the invention is preferably between 0.01% and 99.99% (w/w), more preferably between 0.1% and 60.0% (w/w). In a preferred embodiment, the food biopreservative composition has the following composition (Table 1):

**Table: 5: basic components in the biopreservative composition of the present invention**

| | |
|---|---|
| **Component** | **Concentration Range** |
| bacterial consortium | 1x10⁴ - 1x10⁸ CFU / mL |
| activating agent | 0.01% - 20.0% (w / w) |
| Vehicle (optional) | 0.1% - 99.9% (w / w) |

The biopreservative composition may include other agents such as ascorbic acid, acetic acid or ethanol at concentrations between 0.01 and 10.0%.

In further embodiments, the biopreservative composition has the following composition (Tables 2, 3 and 4):

**Table 6: First non-limiting example of a biopreservative composition of the present invention**

| **Component** | **Concentration** |
|---|---|
| bacterial consortium (*Lactobacillus bulgaricus* and *Streptococcus thermophilus* 1:1) | 9.9 x 10⁶ CFU / mL |
| Cassava starch dispersed in water | 0.3% (w / v) |
| Vehicle (optional) | qs 100.0% |

**Table 7: Second non-limiting example of a biopreservative composition of the present invention**

| **Component** | **Concentration** |
|---|---|
| bacterial consortium (*Lactobacillus bulgaricus, Streptococcus thermophilus* and *Bifidobacterium* 1: 2: 1) | 7.9 x 10⁶ CFU / mL |
| Cassava starch dispersed in water | 0.5% w / v |
| Vehicle (optional) | qs 100.0% |

Table 8: Third non-limiting example of a biopreservative composition of the present invention

| **Component** | **Concentration** |
|---|---|
| bacterial consortium (*Streptococcus thermophilus* and *Bifidobacterium* 2: 1) | 9.9 x 10⁶ CFU / mL |
| Cassava starch dispersed in water | 0.7% w / v |
| Vehicle (optional) | qs 100.0% |

The addition of the biopreservative of the invention was shown to reduce the number of pathogenic bacteria (low number of *Salmonella*, *E. coli*, aerobic mesophilic bacteria) as well as to maintain or improve organoleptic characteristics, such as color and texture of processed food (e.g., hamburger meat), within 48 hours. The biopreservative composition of the invention also allowed inhibiting heat-resistant pathogens such as *Listeria* and enterobacteria.

Similarly, good results were obtained in cooked or scalded sausages contaminated with *E. Coli* O 157: H7, where the reduction of pathogens was higher than 99.0% during the first 100 hours and additionally, in some foods, the composition biopreservative may potentiate the organoleptic behavior. The biopreservative composition of the present invention was also shown to be effective when applied cyclically on fruits and vegetables (e.g., crops of avocado and maize), obtaining fruits free from fungus.

The biopreservative composition may be used in the food industry on a variety of food products such as raw (fresh or dried), processed or canned meat (e.g., poultry, fish, pork, veal, beef, sea food, quarry and bush meat); fresh, dried, processed or canned fruits and vegetables, ready meals (cooked or uncooked) and dry or wet food for pets, including treats. Other non-limiting examples include vacuum packed pâté, hamburger meat and ground meat.

### Definitions:

In order to provide clear and consistent understanding of the terms in the instant application, the following definitions are provided.

The articles "a," "an" and "the" are used herein to refer to one or to more than one (i.e., to at least one) of the grammatical object of the article.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, un-recited elements or method steps and are used interchangeably with, the phrases "including but not limited to" and "comprising but not limited to".

For the recitation of numeric ranges herein, each intervening number there between with the same degree of precision is explicitly contemplated. For example, for the range of 18-20, the numbers 18, 19 and 20 are explicitly contemplated, and for the range 6.0-7.0, the number 6.0, 6.1, 6.2, 6.3, 6.4, 6.5, 6.6, 6.7, 6.8, 6.9, and 7.0 are explicitly contemplated. The terms "such as" are used herein to mean, and is used interchangeably with, the phrase "such as but not limited to".

Unless otherwise defined herein, scientific and technical terms used in connection with the present disclosure shall have the meanings that are commonly understood by those of ordinary skill in the art. For example, any nomenclature used in connection with, and techniques of, cell (including bacterial cell) and tissue culture, molecular biology, bacteriology, immunology, microbiology, genetics and protein and nucleic acid chemistry and hybridization described herein are those that are well known and commonly used in the art. The meaning and scope of the terms should be clear; in the event however of any latent ambiguity, definitions provided herein take precedent over any dictionary or extrinsic definition. Further, unless otherwise required by context, singular terms shall include pluralities and plural terms shall include the singular.

Practice of the methods, as well as preparation and use of the products and compositions disclosed herein employ, unless otherwise indicated, conventional techniques in molecular biology, biochemistry, and microbiology and related fields as are within the skill of the art. These techniques are fully explained in the literature. See, for example, Sambrook et al. Molecular Cloning: A Laboratory Manual, Second edition, Cold Spring Harbor Laboratory Press, 1989 and Third edition, 2001; Ausubel et al., Current Protocols in Molecular Biology, John Wiley & Sons, New York, 1987 and periodic updates; the series Methods in Enzymology, Academic Press, San Diego; and Methods in Molecular Biology, Vol. 119, "Chromatin Protocols" (P. B. Becker, ed.) Humana Press, Totowa, 1999.

As used herein, the term "bacterium" refers not to a single bacterium but to a bacterium population (e.g., a bacterial species). For instance, the term "homofermentative lactic acid bacterium" refers to a "homofermentative lactic acid bacterium" population and mutatis mutandis for "heterofermentative lactic acid bacterium", etc. When a a ratio between two bacterium (i.e., bacterial population) is provided, the ratio refers to the relative amount (e.g., CFU/ml) of each bacterial population.

As used herein, the term "homofermentative LAB" refers to LAB which produce as a primary metabolite lactic acid by the action of the fermentation of a glucose source.

As used herein, the terms "heterofermentative LAB" "heterofermentative bacterium", "heterofermentative lactic acid bacterium" and the like, refer to LAB which produce lactic acid, ethanol and CO₂ as a result of the fermentation of a glucose source. Some *LAB* species are considered "facultatively" heterofermentative, meaning they will produce CO₂ and other by-products only under certain conditions or from specific substrates. These strains would include for example *Lb. plantarum, Lb. casei* and *Lb. curvatus.* Under the context of the present invention, *Streptococcus thermophilus* is heterofermentative. *Lactobacillus* and *Streptococcus* are bacteria used for fermentation processes, which, for the most part, are made under anaerobic conditions. However, in the presence of oxygen, the consortium behaves as an aerotolerant bacteria. In these conditions of stress, *Lactobacillus* stimulates in *Streptococcus* the formation of formic acid, which is a secondary metabolite characteristic of the heterofermentative bacteria. Driessen et al, found that *Streptococcus* produces carbon dioxide, which is necessary for the growth of *Lactobacillus.* This effect is caused by the presence of dissolved oxygen, which changes the growth conditions of the bacterial consortium, *Lactobacillus bulgaricus* and *Streptococcus thermophilus.* This is why *under the context of the present invention Streptococcus* behaves as heterofermentative, producing CO₂, formic acid and lactic acid (see for example, short communication: Effect of oxygen on symbiosis between Lactobacillus bulgaricus and Streptococcus thermophilus, H. Horiuchi and Y. Sasaki) American Dairy Science Association. 2012).

The term "activating agent" for purposes of the present invention is defined as a compound or mixture of compounds that give the bacterial consortium of the biopreservative of the present invention, the energy necessary to activate its enzyme complex and start its growth in a controlled and cooperative manner (regulating the time in each of the phases) and to maintain its metabolic activity. The activating agent should enable the expression of proteases and enzymes (lactate dehydrogenase (LDH), fructose phosphate kinase (PFK), pyruvate kinase (PK) and pyruvic dehydrogenase (PDH)) by homofermentative lactic acid bacteria in the bacterial consortium and the production of formic acid, CO2 and pyruvic acid by the heterofermentative lactic acid bacteria in the consortium. In embodiments, the bacterial consortium in the biopreservative should remain metabolically active for at least 60h, preferably 72h, more preferably 96h and even more preferably 144h. the terms "metabolically active" in the context of the present invention means that the bacteria in the bacterial consortium are in either one of the adaptive, exponential growth or stationary phases for the indicated time (.e., they produce and secrete in the medium lactic acid, CO2, pyruvic acid and/or formic acid and other substances which inhibit the growth or kills bacterial and fungal pathogens such as *E. coli, Salmonella, Aspergillus niger, Aspergillus flavus, Staphylococcus aureus* and/or *Listeria monocytogenes*). In particular embodiments, the activating agent leads to (i) an adaptive phase of between about 1 and 5h, (ii) an exponential growth phase of between about 36-96h (preferably between 48-96h); and a stationary phase of between about 72-144h or more (preferably between 96-144h or more).

The following Examples illustrate the invention.

### EXAMPLE 1: Preparation of a consortium of Lactobacillus bulgaricus and Streptococcus thermophilus from yogurt

From an original yogurt, natural flavor, Alpina brand, 3 sample aliquots were made, 25 ml each. Each aliquot was taken and deposited in tubes containing a buffer solution consisting of 90 ml of 0.1% sterile peptone water (pH 5.7, Difco, Merck KGaA, Germany). Then, circular movements were made in order to homogenize the contents.

A first morphological check was carried out, for which microscopic observations were made, taking one drop of each of the diluted aliquots in the buffer solution of peptone water and carrying out a Gram staining with it. Long bacilli, simple and in strings, with an intense purple color and not sporulated (Gram positive), confirmed the microscopic morphologic characteristics of *Lactobacillus delbrueckii* subsp. *bulgaricus*, and purple cocci grouped in strings (Gram positive), confirmed the microscopic morphologic characteristics of *Streptococcus salivarius* sbsp. *thermophilus.*

Isolation of *Lactobacillus bulgaricus* and *Streptococcus thermophilus.* The isolation of *Lactobacillus* and *Streptococcus* was developed according to the method described by Viloche et al. in 1995, with modifications in the conditions of microaerophilia, temperature and incubation time. From each of the samples prepared from yogurt, decimal dilutions were made in duplicate from 10-1 to 10-4 using the same diluent. Then, 10 petri dishes with MRS agar (MAN, ROGOSA and SHARPE, Merck KGaA, Germany) were prepared and autoclaved.

In the petri dishes with MRS agar, 100 µL of the previously made dilutions were poured and planted using the exhaustion and striation technique. The petri dishes were incubated at 35 °C for 24 hours. After this time, the colonies were analyzed and the typical growth of BAL was observed, white colonies of smooth contours and milky aspect. In order to confirm, a morphological verification with gram staining was made, observing cocci and gram-positive bacilli.

Vitality of the strain isolated from yogurt. From the isolated strains, 5 colonies were taken out of each of the petri dishes and they were planted in tubes with 10 mL of pasteurized milk, in order to verify the bacterial activity and its adaptation to this medium.

The tubes with milk were incubated for 48 hours. After this period, a gram test was performed, observing cocci and gram-positive bacilli, in a ratio of 30:10. When testing the viability of the L. *bulgaricus* and S. *thermophilus* consortium in milk, it was reflected that these maintained a relationship of cooperation to develop in the medium. Lactobacillus grows first and promotes the growth of *Streptococcus.*

Storage and conservation of the L. *bulgaricus* and S. *thermophilus* consortium. The isolated colonies were planted in tubes containing sterile water. Colonies were added to the tubes until obtaining 9.9 x10⁶ CFU, corresponding to 0.28 nephelometric turbidity units (NTU). For this process a MacFarland scale was used, then bacteria were refrigerated at 4 ° C for storage and subsequent lyophilization.

### EXAMPLE 2: Preparation of a biopreservative composition comprising L. bulgaricus and S. thermophilus

Initially 0.75 g of cassava starch were dispersed in 250 ml of sterile water (0.3% solution) and the obtained dispersion was autoclaved for 30 minutes at 76 ° C +/- 2. Subsequently, 0.62 g (about 9.9 x 10⁶ CFU) of lyophilized lactic acid bacteria of a mixture of *L*. *bulgaricus* and S. *thermophilus* in a ratio of 1:1, obtained according to Example 1 were taken and added in 10 mL of sterile dispersion comprising cassava starch to obtain a biopreservative composition.

### EXAMPLE 3: Cooperation between Lactobacillus bulgaricus and Streptococcus thermophilus

The cooperative relationship between *Lactobacillus bulgaricus* and *Streptococcus thermophilus* was studied as follows.

**Obtaining the equivalency in CFU from the commercial culture.** Strains of microorganisms were obtained from commercially available sources (SACCO Lyofast Y 450 B x 5 UC). The commercial culture comes in a 5 grams' presentation. To obtain a concentration in CFU from the commercial culture, 2.5 grams and 5 grams were taken and dilutions of 10⁵ and 10⁶ were prepared in distilled sterile water. These dilutions were planted using the pour plate technique in MRS agar, by triplicate, and they were incubated to 37°C for 24 hours.

Once the incubation time elapsed, counting was performed for each of the petri dishes and the results were averaged in order to know the relation between grams of commercially available culture and the amount of CFU. After adaptation, planting and incubation of commercial culture, dilutions were made.

Following incubation, a recount of the CFU that grew in each of the tested concentrations was made. The results are reported in the following table.

**Table 9: CFU equivalence from a commercially available culture**

| **Dilution** | **Amount of commercial culture** | **Average of recount CFU of LAB** |
|---|---|---|
| 10⁵ | 2.5 g of the commercial culture | 9.9 x 10⁵ CFU |
| 10⁶ | 2.5 g of the commercial culture | 28.1 x 10⁶ CFU |
| 10⁵ | 5 g of the commercial culture | 56 x 10⁵ CFU |
| 10⁶ | 5 g of the commercial culture | 56.2 x 10⁶ CFU |

**Formulation of biopreservative concentrations, determination of growth and utilization of substrate.** 3.9 x 10⁶ CFU/ml were inoculated in 10 ml of 0.1% starch solution (prepared using 0.2 g of cassava starch in 200 ml of sterile distilled water-hereinafter biopreservative B). Also, 9.9 x 10⁶ CFU were inoculated in 10 ml of 1% starch solution (hereinafter, biopreservative A). Biopreservatives A and B were incubated at 37 °C for 24 hours.

Next, analysis of *Lactobacillus bulgaricus* and *Streptococcus thermophilus* viability and growth (cooperation) characteristics in a biopreservative was performed.

After 24 hours of incubation of a bacterial consortium comprising *Lactobacillus bulgaricus* and *Streptococcus thermophilus* (in a ratio of 1:1), tests were carried out to determine the growth characteristics of the consortium, the use of the starch as a substrate and the morphology of bacteria present in the biopreservative. The tests performed included pH determination, according to the method 981.12 of the A.O.A.C. (Association of official analytical chemists - A.O.A.C) (1990), bacterial count, (according to the plate count method), substrate consumption by determination of reducing sugars (3,5-dinitrosalicylic acid method - DNS), lactic acid production according to method 947.05 of A.O.A.C. (1990) and Gram stain.

**Table 10: CFU in commercial culture**

| **Biopreservative** | **Amount of commercial culture** | **CFU of LAB** | **Amount of 0.1% starch solution** | ***DNS*** | ***pH*** |
|---|---|---|---|---|---|
| Biopreservative **A** | 0.62g of the commercial culture | 9.9 x 10⁶ CFU | 10 ml | 43.23 | 5,78 |
| Biopreservative **B** | 0.35g of the commercial culture | 3.9 x 10⁶ CFU | 10 ml | 22.65 | 5,49 |

Following Gram staining, Gram-positive bacilli and cocci grouped in strings were observed, in ratio 1:1. Biopreservative A was able to efficiently adapt to the substrate, being able to unfold it and assimilate it as a carbon source. This result is reflected in the highest concentration of reduced sugars in the DNS test. The above findings indicate that biopreservative A has an optimal formulation.

*Lactobacillus delbrueckii* subsp. *bulgaricus* and *Streptococcus thermophilus* are traditionally used in fermented foods. These two species interact in a growth promoting mutual benefit through the exchange of metabolites, a process known as protocooperation.

The activating agent selected for use in the biopreservative of the present invention supports the synergy between the heterofermentative and homofermentative bacteria in the bacterial consortium. The bacterial consortium in the biopreservative of the present invention generally has the following growth phases. First, there is an adaptive phase (stationary phase) during which lactic acid, formic acid, CO2 and exopolysaccharides are not produced (or produced in insignificant amounts). Following the adaptive phase, there is a growth phase, where lactic acid, formic acid, CO₂ and exopolysaccharides are produced by the bacterial consortium. It is during the growth phase that most lactic acid is produced, mainly by the homofermentative bacteria (e.g., lactobacillus). The number of bacteria in consortium rapidly increases during this phase (e.g., from 8x10² to 1,6 x10⁶). Table 11 below shows the amounts of lactic acid, formic acid, CO₂ and exopolysaccharides which are produced by the bacterial consortium during the growth phase. The last phase is the stationary phase during which bacterial growth significantly slows down or stagnates. During this phase, lactic acid, formic acid, CO₂ and exopolysaccharides produced by the bacterial consortium continue to accumulate in the medium.

Table 11 below shows an example of bacterial growth kinetics (growth phases) using biopreservative A described above

**Table 11: Bacterial growth kinetics of biopreservative A**

| **Growth phases *Lactobacillus* and Streptococcus** | **Time (h)** | | **Lactic acid (%)** | | **Formic acid (%)** | | **CO₂ (%)** | | **pH** | | **EPS* (%)** | | **CFU** | |
|---|---|---|---|---|---|---|---|---|---|---|---|---|---|---|
| | Mi n | Max | Min | Max | Min | Max | Min | Max | Min | Max | Min | Max | Min | Max |
| **Adaptation** | 1 | 5 | NO | NO | NO | NO | NO | NO | 5.3 | 5.4 | NO | NO | 8.00E+00 | 9.00E+01 |
| **Growth** | 48 | 96 | 0,3 | 0,4 | 0,2 | 0,3 | 1 | 2 | 5.5 | 5.7 | 2 | 3 | 8.00E+02 | 1.60E+06 |
| **Stationary** | 96 | 144 | 0.7 | 0.12 | 0.3 | 0.5 | 3 | 5 | 5.19 | 5.2 2 | 4 | 7 | 1.80E+06 | 2.60E+07 |

| | | | | | | | | | | | | | | |
|---|---|---|---|---|---|---|---|---|---|---|---|---|---|---|
| *exopolysaccharides | | | | | | | | | | | | | | |

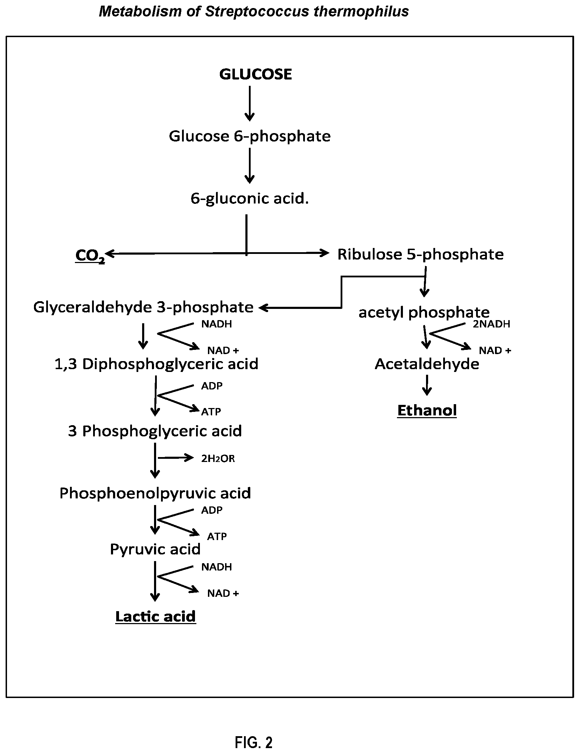

*Streptococcus* (an heterofermentative LAB) grows first in the growth medium and produces formic acid, pyruvic acid and carbon dioxide (FIG. 2), which are metabolites necessary for growth of *Lactobacillus. Lactobacillus,* in turn expresses extracellular proteases in the presence of food (energy sources) containing amino acids and proteins. In this process small peptides are released which are metabolized from peptides to amino acids, which allows *Streptococcus* to continue its growth in the medium. The expression of: lactate dehydrogenase (LDH), fructose phosphate kinase (PFK), pyruvate kinase (PK) and pyruvic dehydrogenase (PDH) by *Lactobacillus,* allows it to transform multiple substrates into an energy source for *Streptococcus.*

This cooperative relationship produces a competition for the use of nitrogen and carbon sources available in the medium, which stimulates the growth rate of *Lactobacillus* and *Streptococcus.* As result of this consortium, bactericides substances such as lactic acid, formic acid, CO₂ and pyruvic acid, are released, which effectively inhibit pathogenic bacteria present in complex proteic materials such as, pork, chicken and sausages, as shown in Examples 4 and 6 below.

### EXAMPLE 4: Inhibition of E. coli 0157: H7 in pork samples

To a biopreservative composition obtained according to Example 2 were performed growth inhibition assays of microorganisms in pork samples contaminated with *E*. *coli* O157: H7. The experiments were conducted as follows. Preparation, isolation and verification of *E*. *coli* O157: H7 strain. The *E*. *coli* O157: H7 strain was supplied by the biotransformation group of the University of Antioquia (Medellin, Colombia). For the control of the strain, biochemical analyzes were carried out, described in the manual of procedures, diagnostics and characterization of *Escherichia Coli* 0157 H: 7 production of Shiga toxin from foods Rivas et al. (2008) A commercial immunochromatographic test (NEOGEN Corporation) was also performed to confirm the serotype of the microorganism.

Preparation of a biopreservative composition. 9.9x10⁶ CFU of the *Lactobacillus bulgaricus* and *Streptococcus thermophilus* consortium were taken and added in three tubes containing 10 ml of 0.1% cassava starch solution. The tubes containing the biopreservative were incubated at 37 °C for 24 hours.

Preparation of *E*. *coli* O157: H7. Four colonies of *E*. *coli* O157: H7 were taken and suspended in three tubes containing 10 ml of 0.85% normal saline solution. This solution contained 1x10⁸ CFU of *E*. *coli* O157: H7. Concentration of *E*. *Coli* was confirmed with a Turbidimeter series 100Q / 2100QIS from Hach™. Tubes comprising the suspension of the pathogen were incubated at 37 °C for 24 hours.

Preparation of pork samples. Three completely sterile glass containers were prepared. In each container, 45 g of ground pork and 190 ml of distilled water were added.

Addition of pathogenic microorganism. The pork samples were contaminated by adding the content of tubes previously prepared with the pathogenic *E*. *coli* O157: H7 strain, to each container (one tube per container). Containers comprising the contaminated pork samples (as well as control samples) were brought to refrigeration at 4 °C for 1 hour.

Addition of biopreservative. The contaminated containers were removed from the refrigerator, after which two tubes containing the previously formulated biopreservative were added, one tube per container. The containers were next refrigerated at 4 ° C for 144 hours. A contaminated sample comprising no biopreservative was used as a reference for the growth of *E*. *coli* O157: H7 in the pork sample.

Measurements. Samples were taken every hour for pH determination, according to method 981.12 of the A.O.A.C. (1990), BAL count (as per plate count method), substrate consumption by determination of reducing sugars (3,5-dinitrosalicyclic acid method - DNS) and commercial immunochromatographic test for *E*. *coli* O157: H7.

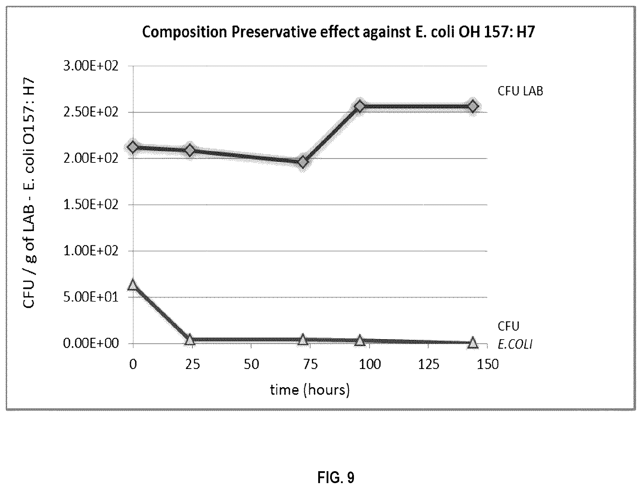

The biopreservative composition inhibited the growth of *E*. *coli* O157: H7 in pork meat samples having a concentration of 1x10² UFC of infectious dose of the pathogenic agent. As illustrated in FIG 9, a decrease in the number of *E*. *coli cells* is observed within 24 to 72 hours. After 72 hours, the decrease in the concentration of pathogen is notorious thanks to an increased in the production of antimicrobial substances exposed in food by the biopreservative during its exponential growth phase.

### EXAMPLE 5: Inhibition of Aspergillus flavus and Aspergillus niger on fruits and vegetables

Biopreservative A (see Example 4) was also tested for the inhibition of *Aspergillus flavus* and *Aspergillus niger* on corn and avocado.

Avocado and corn were collected in the municipality of Carmen de Bolivar, located in the department of Bolivar, Colombia. In avocado-producing and maize-producing farms, products having the following macroscopic appearance were picked: Avocados with presence of black spots, soft areas and bad smell. Maize, with presence of green spots, earthy texture, with or without bad smell.

After having selected the avocados, microbiological isolation was carried out by removing the affected tissue from the avocados, and adding it in a 70% ethanol solution for 5 minutes. Samples were then allowed to dry on absorbent paper for 2 minutes. Subsequently, each of the tissue samples were placed in Petri dishes with Sabouraud Agar (Merck, Millipore), and incubated at room temperature. After 48 hours, reading was performed in order to obtain the microscopic and macroscopic study of *Aspergillus niger.*

After having selected the affected maize, the cob was removed and each corn was striped with the help of sterile knives. Ten grams of these samples were deposited in Ziploc® bags labeled as Sample 1 to Sample 12. They were then macerated in a Stomacher lab blender (BagMixer™, interscience) for 60 seconds. Subsequently, each of the samples was placed in screw cap tubes containing 90 ml of peptone water and then serial dilutions of 10¹ to 10⁸ were made. These dilutions were plated on petri dishes with potato agar (Merck, Millipore) and subsequently incubated at room temperature for 7 days. After this time, mycotic strains with growth characteristics of *Aspergillus flavus* were found. From the obtained growth a staining with blue of lactophenol was made for the microscopic identification of the fungus. It was observed that structures compatibles with *Aspergillus flavus* were present.

Preparation of suspension *Aspergillus niger* and *Aspergillus flavus.* This step was necessary in order to determine the concentration of fungus. Briefly, eight (8) sterile tests tubes with 9 ml of distilled water were taken, each marked with their respective dilution from 10¹-10⁸. A small part of the *Aspergillus niger* culture was then taken and introduced into the first test tube. It was immediately homogenized and from each tube it was extracted 1ml until obtaining a dilution of 10⁸. With this dilution, planting was carried out in 10 petri dishes of agar Sabouraud. This same procedure was performed with the maize samples for *Aspergillus flavus* on potato agar. All samples had an initial concentration of 1x10⁸ CFU. The petri dishes were incubated at room temperature for 48 hours.

Separately, 10 petri dishes of *Aspergillus niger* 1x10⁸ CFU and 10 petri dishes of *Aspergillus flavus* 1x10⁸ CFU were taken and incubated under the same conditions as the above as control for the fungus growth.

### Application of the biopreservative.

First, blotting paper strips were made. Subsequently, each strip was submerged in tubes comprising the biopreservative as described in Example 4. The strips were deposited in petri dishes of Sabouraud Agar previously planted with the fungus. In each petri dish, two bands were deposited clockwise, marking the 6 o'clock. This method was described by the National Committee for Clinical Laboratory Standards (NCCLS, also known as the Clinical and Laboratory Standard institute (CLSI), Standards. 1997. Reference method for broth dilution antifungal susceptibility testing of yeasts. M-27A. National Committee for Clinical Laboratory Standards, Villanova, Pa.).

The number of colonies present in each of the petri dishes, in which the biopreservative was applied, was counted. From this data, an average was calculated.

The biopreservative achieved the inhibition of fungi that contaminate avocados and corn such as *Aspergillus flavus* and *Aspergillus niger.* The findings of the invention confirm that the biopreservative can stop the growth of the above-mentioned fungi, after 24 hours of application of the biopreservative.

**Table 12: Inhibition of Aspergillus flavus and Aspergillus niger on corn and avocado (Haas variety) using a biopreservative composition as described in Example 4. Please see the table.**

| **FUNGI** | **with biopreservative** | | **No biopreservative** | |
|---|---|---|---|---|
| | 24 (h) | 48 (h) | 24 (h) | 48 (h) |
| *Aspergillus flavus* in corn | 1.00E + 08 | 1.00E + 05 | 1.00E + 08 | 1.00E + 11 |
| *Aspergillus niger* in avocado | 1.00E + 08 | 1.00E + 06 | 1.00E + 08 | 1.00E + 09 |

The biopreservative was shown to efficiently inhibit the growth of fungi (*Aspergillus flavus* and *Aspergillus niger*) that contaminate fruits and vegetables such as corn and avocado. The biopreservative stopped the growth of fungi, after 24 hours of application of the biopreservative.

### EXAMPLE 6: Inhibition of pathogenic microorganisms in pork, chicken and sausage samples

Growth inhibition assays on pork, chicken and sausage samples were performed using a biopreservative composition obtained according to Example 2 (1:1 *Lactobacillus delbrueckii* subsp. *bulgaricus*: *S. thermophilus*). The samples were contaminated with 1x10⁸ CFU of *E*. *coli* O157: H7 or 1x10³ CFU of *E*. *coli* (*Enteropathogenic,* extracted from contaminated cow meat), 1X10⁵ CFU of *Staphylococcus aureus*, 1x10⁶ CFU of *Salmonella*, or 1x10⁹ CFU of *Listeria monocytogenes.*

Preparation of biopreservative. For this test, three tubes with biopreservative, comprising 10 ml of 0.1% starch solution and 0.62g of commercial culture (SACCO Lyofast™ Y 450B x 5 UC) were prepared. Each of the tubes was sealed and incubated at 37 °C for 24 hours. After 24 hours Gram staining was performed to verify the presence of the two morphologic characteristics of the consortium (Cocci and Gram-positive bacilli), in a ratio of 1:1. The tubes with biopreservatives were submerged in a tank with liquid nitrogen, in order to freeze the whole contents of the tubes. After the freezing stage, the tubes with biopreservative were lyophilized, using a LABCONCO lyophilizer with the following conditions; Temperature -45 ° C, vac: 0.021, time 27 hours. As a result of this process, the biopreservative was obtained in powder and kept at -1 °C, until use.

Preparation of pathogenic microorganisms. The pathogenic strains were supplied by the biotransformation research group of the University of Antioquia (Medellin, Colombia). Growth inhibition of the following pathogens were tested: *E*. *coli* enterotoxigenic, *Staphylococcus aureus, Salmonella spp, Listeria monocytogenes* and *E*. *coli* O157H: 7. For the preparation of the microorganisms, planting was made in selective mediums for each genus. Representative colonies of medium size were then taken and immersed in tubes with 0.85% saline solution. For each microorganism, four (4) tubes were prepared. For each microorganism, the concentration selected was the minimum dose at which the pathogen can cause a disease in the host. The concentrations were as follows; *E*. *coli* enterotoxigenic 1x10³ CFU, *Staphylococcus aureus* 1x10⁵ CFU, *Salmonella* spp 1x10⁶ CFU, *Listeria monocytogenes* 1x10⁹ CFU and *E*. *coli* O157H:7 1x10⁸ CFU. The concentration of each the pathogen was measured by turbidimetry using a turbidimeter series 100Q / 2100QIS from Hach™. All preparations of pathogenic microorganisms were kept at -1 °C, until use.

Meat sources. The meat matrices selected for this trial were pork, chicken and sausages. They were bought at a certified supermarket. With each of the matrices, adapted food formulations were made. As follows:

Pork. Two loins of pork, 2kg each, were taken, cut in pieces 2 to 3 cm and placed in Ziploc® gallon /large bags. They were then macerated until reaching a grain size of about 4 to 5 millimeters. The maceration was performed using a stomacher equipment brand (BagMixer™, Interscience) for 15 minutes. To obtain a more manageable pork paste, 150 ml of sterile water were added, after which one of the bags was inoculated with the biopreservative and each of the pathogenic bacteria. After this, the bag was sealed and homogenized manually. In the second bag neither pathogenic bacteria nor biopreservative were added, as a control. The bags were left in refrigeration in a refrigerator (Distrinordico) at 3.5°C, during 144 hours.

Chicken. Two boneless chicken breasts, each weighing 2 kg, were cut in pieces of 2 to 3 cm and placed in Ziploc® gallon/large bags. They were then macerated until reaching a grain size of about 4 to 5 millimeters. Maceration was performed using a Stomacher lab blender (BagMixer™, Interscience) for 15 minutes. One of the bags was inoculated with the biopreservative and each of the pathogenic bacteria. After this, the bag was sealed and homogenized manually. In the second bag neither pathogenic bacteria nor biopreservative were added, as a control. The two bags were left in refrigeration in refrigerator (Distrinordico) at 3.5°C for 144 hours.

Sausages. Two batches of sausages of 2kg each were made. The use of nitro salt was replaced by the application of the biopreservative, representing 0.4% of the ingredients in each of the formulas. One batch was inoculated with the biopreservative and each of the pathogenic bacterial species, this process was performed in the mixing stage. Another batch was left without biopreservative and without pathogenic bacteria as control. For the production of sausages, the basic steps for the elaboration of a raw sausage were followed. The sausages were left in refrigeration in a refrigerator (Distrinordico) at 3.5°C for 144 hours.

Evaluation of microorganisms present in pork, chicken and sausage. Samples were taken hourly from the different meat formulations during 144 hours. In each of the samples, the presence of inoculated pathogenic microorganisms was evaluated, according to the method described in the Manual of Analytical Bacteriology of the Food and Drug Administration (FDA) (Hitchins, 1995), with variations in the detection of *E*. *coli* O157: H7, which was performed with the immunochromatographic test (NEOGEN Corporation) described in Example 4.

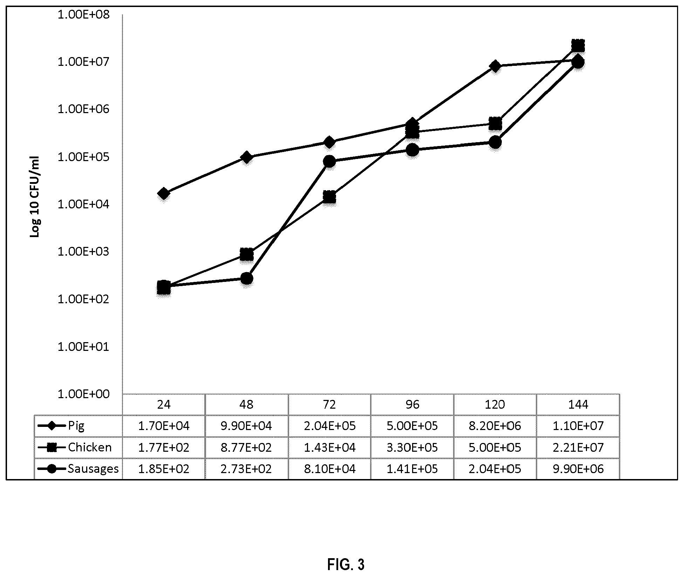

The lactic acid bacteria present in the biopreservative were measured using the method of viability evaluation of *Lactobacillus bulgaricus* and *Streptococcus thermophilus* as a biopreservative described in Example 1. See results in FIG. 3.

As shown in Table 13 below, the biopreservative effectively inhibited the growth of pathogenic bacteria in all sample tested. As in Example 5, a decrease in pathogenic bacteria is observed within hours. After 48 hours, complete decontamination of pathogens achieved, thanks to an increase in the production of antimicrobial substances exposed in food by the biopreservative during its exponential growth phase.

**Table 13: Inhibition of pathogenic bacteria using a biopreservative composition comprising Lactobacillus bulgaricus and Streptococcus thermophilus (1:1) in a 0.1% starch solution.**

| **Pathogenic bacteria** | **24 h** | | **48 h** | |
|---|---|---|---|---|
| | No biopreservative | W/biopreservative | No biopreservative | W/biopreservative |
| ***E. coli** (Enteropathogenic,* extracted from | 75% | 0% | 75% | 0% |
| contaminated cow meat) | | | | |
| ***Staphylococcus aureus*** | 5% | 0% | 0% | 0% |
| ***Salmonella*** | 50% | 12% | 40% | 0% |
| ***Listeria monocytogenes*** | 100% | 20% | 100% | 0% |
| ***E. coli O157 H:7*** | 30% | 10% | 40% | 0% |

### EXAMPLE 7: Carbon sources as activating agents

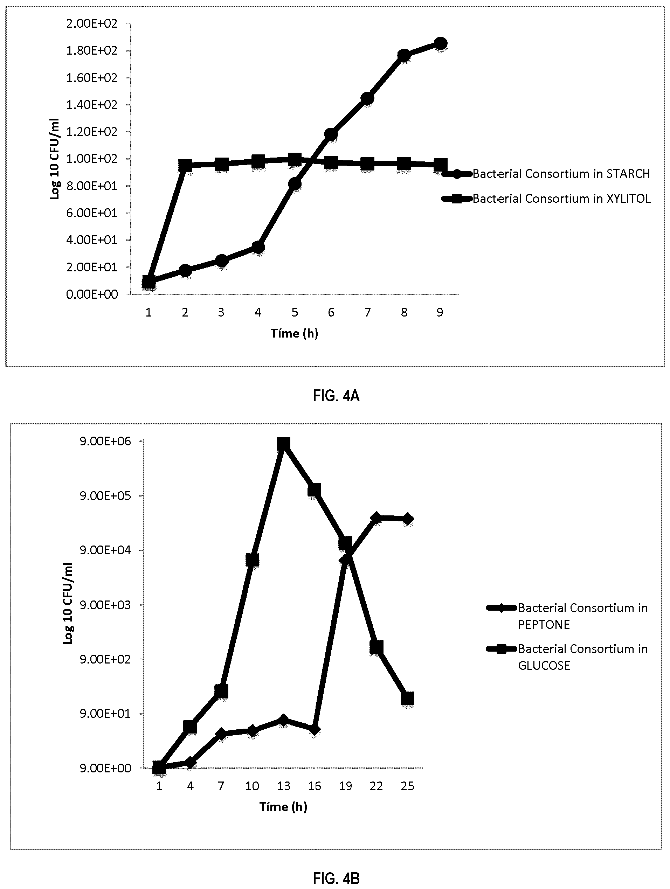

Various substrates (cassava starch, xylitol, glucose and peptone) were tested as carbon sources for a bacterial consortium comprising *Lactobacillus delbrueckii* subsp. *bulgaricus* and *Streptococcus thermophilus* (in a ratio of 1:1) were prepared in Erlenmeyers as follows: solutions of xylitol 1.5% (w/v), glucose 1.0% (w/v), 0.1% (w/v) cassava starch and 1.2% (w/v) peptone in 250 ml sterile water. Next, 10 ml of the liquid biopreservative described in Example 4 were added in each Erlenmeyer. Samples were taken every hour to analyze the growth of the biopreservative in the carbon sources, applying the method of plate count. Results are presented in FIG. 4A and B.

Xylitol, C₅H₁₂OR₅, is a five-carbon sugar alcohol. It may be used by certain homofermentative bacteria such as *Lactobacillus delbrueckii subsp. Bulgaricus*, thanks to the enzymatic package that it possesses, which is capable of reducing xylitol to D-xylulose (a D-ketose that belongs to the 5 carbon atoms series of monosaccharides). As shown in FIG. 4A, metabolic activation of the consortium is possible using xylitol as a source of carbon. Indeed, the bacterial consortium was shown to have an optimal development in each of its stages of growth (FIG. 4A).

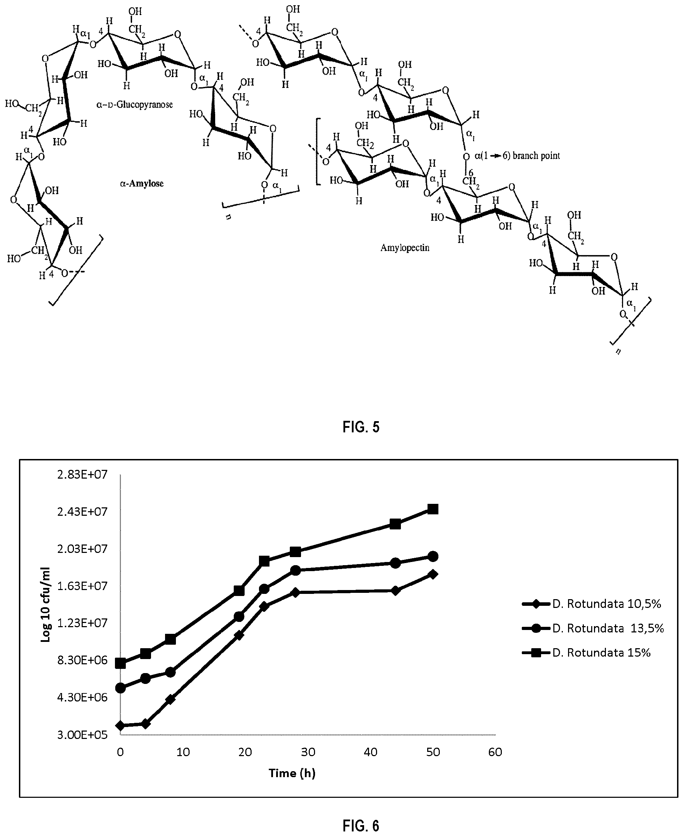

Cassava starch is a complex and closed molecule, composed of two monomers of glucose: amylose and amylopectin. It is characterized by a three-dimensional helical buckling structure of the polymer in the link β(1-4). Amylopectin has in its structure between 2000 to 200,000 units of glucose (see FIG. 5 for structure). Amylose has between 300 to 350 units of glucose.

As shown in FIG. 4A, adaptation of the bacterial consortium to Cassava starch was highly effective. Cassava starch was rapidly recognized as a primary source of energy and promoted metabolic activation, using the glucose required for each of the growth phases. Use of Cassava starch led to an increase in the speed of adaptation of the consortium in the food and to the expression of primaries and secondary metabolites, thereby sustaining metabolic activity of the consortium quickly and for a longer period of time.

As shown in FIG. 4A, the first growth phase is slower when starch is used as source of carbon as when xylitol is used. Such effect may be due to the fact that in order to access the glucose available in the starch, the bacterial consortium must first open the structure by cleaving the links (leaving available the carbon source for its metabolism). However, once glucose is accessed, metabolic activation becomes highly effective.

Glucose (C₆H₁₂OR₆) is a 6 carbons open molecule, which is directly accessible and rapidly metabolized by the bacterial consortium in the first growth phase. As shown in FIG. 4B, glucose is rapidly metabolized by the consortium and is unable to sustain its growth in a second growth phase (cooperation between the bacteria in the consortium does not occur). The number of bacterial cells in the consortium started declining rapidly within 10-12 hours and reached death within 48 hours. These results show that glucose does not support the synergistic behavior of the consortium as it is unable to sustain a second growth phase as shown, for example for starch.

Corresponding results are expected with Dextrose (C₆H₁₂OR₆, the D-isomer or glucose) because, just as glucose, it has a completely open structure, making it readily available for quick use by the bacteria.

Peptone, is rich in protein and thus comprises less carbohydrates than starch or other carbon sources. As a consequence, the growth of the bacterial consortium using such energy source was slow, delaying to 16 hours the adaptation stage of the consortium (FIG. 4B). It follows that the production of primary metabolites began about 24 hours after application of the biopreservative. These results indicate that peptone is not a good substrate for the consortium, because pathogenic bacteria would have too much time to grow before they are killed by the secondary metabolites in the second growth phase.

### EXAMPLE 8: Yam and plantain peels as activating agents

Yam and plantain peels were also tested as activating agents for the consortium of the present invention.

Briefly, 1.34kg of variety of yam *D. rotundata.* were peeled, then washed with a solution of 1M ammonia in order to eliminate the mucilaginous residue of the tuber. They were then cut into pieces of 3 to 5 cm, to be subsequently processed in a food processor (black and decker), for 5 to 10 minutes. Followed to this, drying was carried out in a BRINDER oven at 300°C, during 15 minutes. After this time, samples were ground (IMUSA manual mill), to obtain smaller particles and a texture of powder. The yam starch was packed in Ziploc™ bags (Method described by Azturizaga et al. 2008).

Three solutions of yam were prepared, using a commercial amyloglucosidase enzyme, which was added to three Erlenmeyers containing 3 g of yam starch in 250 ml of sterile water (a 0.3% solution). The volume of applied enzyme are as follows 10.5 ml, 13.5 ml and 15 ml.

Prior to this, each Erlenmeyer was inoculated with the biopreservative, prepared as indicated in Example 4 and the growth of the bacterial consortium in the presence of yam starch analyzed.

Also, extracts from plantain (green banana) peels were prepared and tested as a carbon source for the consortium as follows.

Briefly, 4 kg of banana peels (shells) contained in Ziploc™ bags for conservation purposes were used and stored in refrigeration using a Samsung refrigerator at a temperature of 18 °C. The banana shells were washed with Pursue™ disinfectant solution and cooked at a temperature of 100 °C for 45 minutes. They were then cut into small pieces of approximately 2 x 2 cm and were crushed in a Black and Decker processor for 5 minutes. From the above, a banana shell substrate was obtained, from which 45 grams were weighed and added to three (3) 300ml containers of sterile distilled water to obtain a concentration of 15% (w/v).

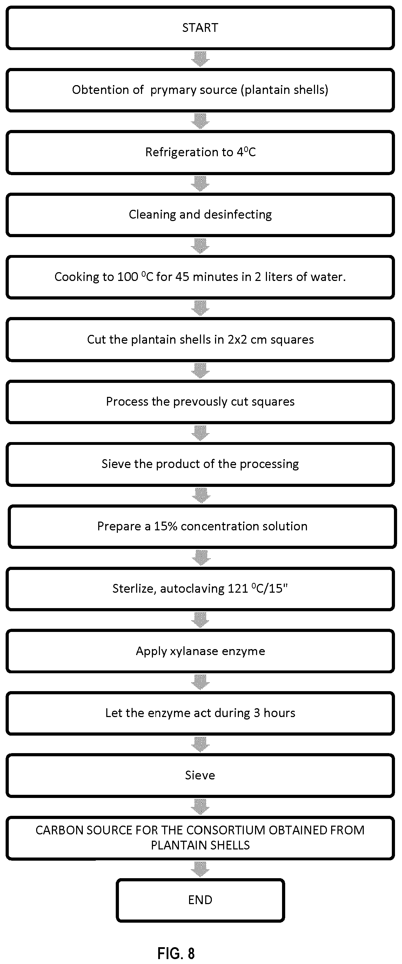

These containers with substrates were autoclaved. To the prepared and sterile substrates were added 6 ml of xylanase enzyme. The substrates were allowed to stand for a time of 3 hours. (Procedure described by Green Vineyards M et al., 2012). An overview of the process used to prepare starch from plantain peels (also referred as shells) is shown in FIG. 8.

The effect of plantain peels extract (15%) on the growth of a bacterial consortium comprising *Lactobacillus bulgaricus* and *Streptococcus thermophilus* (1:1, 9.9x10⁶ CFU) was studied. Samples were taken for 84 hours and lactic acid bacteria were counted. Gram staining confirmed the presence of bacilli and gram positive cocci in a 1:1 ratio.

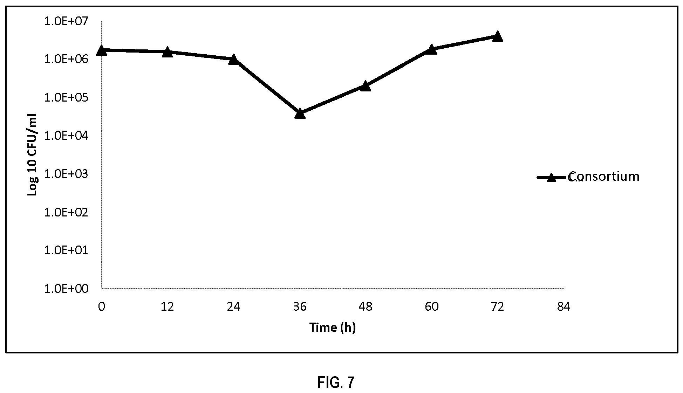

Results for the use of yam as a source of carbon are presented in FIG. 6. Results for the use of a plantain peel extract as a source of carbon are presented in FIG. 7.

Taken together, Applicant studies have shown that, the types of starch that is preferably used by the bacterial consortium of the present invention has an amylose content higher than or equal to 17% (w/w) or a content of amylopectin lower than or equal to 83% (w/w) at a ratio of 17:83 of amylose: amylopectin but which preferably does not exceed a ratio of 24:76 amylose: amylopectin.

### EXAMPLE 9: Organoleptic features of food products

To determine the effect of the biopreservative on the flavor of the sausages, an acceptance test was carried out with a panel of 50 untrained individuals. After 144 hours of incubation of the sausages with or without biopreservative, the sausages were cooked for 7 minutes in a pan at 37 °C +/- 2, (see Anzaldúa, 1998, Leon et al., 2006).

Testers first tasted the sausages that did not contain the biopreservative, after which they took 10 ml of water, before continuing with the sausages that contained the biopreservative.

Each person evaluated the attributes of color, flavor and texture using a nine-point hedonic scale with extremes 9 "extremely liked" and 1 "extremely disagreeable". All the results were recorded in a format of answers that were later tabulated for analysis.

**Table 14: Consumers impression of food products comprising a biopreservative as described in Example 6**

| | **with biopreservative** | **No biopreservative** |
|---|---|---|
| **COLOR** | 72% | 72% |
| **TEXTU RE** | 87% | 75% |
| **FLAVOR** | 98% | 77% |

The improvements in texture and flavor reported following consumer testing is thought to be due to the secretion by *Lactobacillus* of moderate concentrations of acetaldehydes, due the action of *Streptococcus.* Acetaldehydes improved the organoleptic characteristics in food such as, flavor and aroma. As shown above, in meat products treated with a biopreservative as described in Example 6, the consumer acceptance was close to 100%.

### REFERENCES

1. Ricardo Adolfo Parra Huertas. Review. Lactic acid bacteria: functional role in food. Faculty of Agricultural Sciences. Vol 8 No 1. January- July 2010.
2. Samaniego Fernandez, Sosa del Castillo. Lactobacillis spp: importantes promotores de actividad probiótica, antimicrobiana y bioconservadora. Centro de estudios biotecnológicos. Facultad de Agronomia. Universidad de Matanzas "Camilo Cienfuegos". Matanzas, Cuba;
3. Meneses, Corrales y Valencia. 2007. Sintesis y caracterización de un polimero biodegradable a partir de almidón de yuca. Escuela de Ingenieria. Revista EIA ISSN 1794-1237 No 8 p57-57 diciembre 2007.
4. Short communication: Effect of oxygen on symbiosis between Lactobacillus bulgaricus and Streptococcus thermophilus, H. Horiuchi and Y. Sasaki) American Dairy Science Association (2012);
5. Asturizaga Avilez, Yajaira and Bocanegra Amaya, Carmen. Evaluation of yields in the process of obtaining alcohol from yam flour (Dioscorea Bulbifera, Trifida) by enzymatic route. Sincelejo. Universidad de Sucre. Faculty of Education and Sciences. Biology Program, 2008. p. 108.
6. Green Vineyards M, Bell Garcia A, Michelena Alvarez G, Ramil Mesa M. Obtaining Bioethanol from Lignocellulosic Biomass ICIDCA. About Sugar Cane Derivatives [online] 2012, 46 (January-April).
7. Anzaldúa, A. (1998). Evaluación sensorial de los alimentos en la teoria y en la práctica. Editorial. Acribia S.A. Zaragoza España. P. 214. Sensory evaluation of food in theory and practice. Editorial. Acribia S.A. Zaragoza Spain.
8. Leon V, Totosaus A, Guerrero, I, Pérez, M (2006). Efecto de bacterias ácido lácticas termoresistentes en salchichas cocidas. Revista Ciencia y Tecnologia Alimentaria. 5: (002)135-141.
9. Viloche Bazán Juan, Tito Vargas. (2007). Aislamiento de Lactobacillus nativos de productos de fermentación en la ciudad de tacna. Revista Ciencia y Desarrollo, COIN-UNJBG. No 11. Pag. 61-66. [online] Isolation of native Lactobacillus from fermentation products in the city of tacna. Science and Development Magazine.
10. Rivas, Marta. Leotta, Gerardo. (2008) Manual de Procedimientos. Diagnóstico y caracterización de Escherichia coli 0157 productor de toxina Shiga a partir de alimentos . Chinen, Isabel. Departamento Bacteriologia Instituto Nacional de Enfermedades Infecciosas A.N.L.I.S. "Dr. Carlos G. Malbrán" Centro Regional de Referencia del WHO Global Salm Surv para América del Sur. P147.
11. National Committee for Clinical Laboratory Standards. 1997 (NCCLS). Reference method for broth dilution antifungal susceptibility testing of yeasts. M-27A. National Committee for Clinical Laboratory Standards, Villanova, Pa.
12. Official Methods of analysis. (1990). 15 edition Edited by Kenneth Helrich, published by the Association of official analytical chemists, Inc. Suite 400 2200 Wilso Boulevard, Arlington. Virginia 2201 USA.

## Claims

1. A food biopreservative composition comprising (i) a consortium of lactic acid bacteria comprising at least one homofermentative lactic acid bacterium and at least one heterofermentative lactic acid bacterium; and (ii) an activating agent, wherein the activating agent is starch, a carbohydrate having from 3 to 5 carbon atoms or comprises a fructooligosaccharide and is either in solid form, in solution or dispersion in a concentration of between 0.001% and 20.0% (w/w) in the composition, and wherein
(a) the homofermentative lactic acid bacterium is *Lactobacillus delbrueckii* subsp. *bulgaricus, Lactobacillus delbrueckii* subsp. *lactis, Lactobacillus delbrueckii subsp. leichmannii, Lactobacillus acidophilus, Lactobacillus amylophilus, Lactobacillus amylovorus*, *Lactobacillus animalis, Lactobacillus crispatus, Lactobacillus farciminis*, *Lactobacillus gasseri, Lactobacillus jensenii, Lactobacillus ruminis, Lactobacillus salivarius, Lactobacillus sharpeae*, *Lactobacillus vitulinus, Lactobacillus yamanashiensis* or any combination of at least two thereof; and/or (b) the heterofermentative bacterium is *Streptococcus thermophilus, Leuconostoc, Pediococcus, Lactobacillus plantarum, Lactobacillus rhamnosus, Lactobacillus helveticus* or any combination of at least two thereof.

2. The food biopreservative composition of claim 1, wherein:
(i) the at least one homofermentative lactic acid bacterium produces proteases; and the at least one homofermentative lactic acid bacterium and the at least one heterofermentative lactic acid bacterium are in a ratio (1:1);
(ii) the at least one homofermentative lactic acid bacterium produce proteases; and the at least one homofermentative lactic acid bacterium and the at least one heterofermentative lactic acid bacterium are in a ratio (2:1);
(iii) the at least one homofermentative lactic acid bacterium produce proteases; and the at least one homofermentative lactic acid bacterium and the at least one heterofermentative lactic acid bacterium are in a ratio (1:2);
(iv) the at least one homofermentative lactic acid bacterium is a probiotic lactic acid bacterium; and the at least one homofermentative lactic acid bacterium and the at least one heterofermentative lactic acid bacterium are in a ratio (1:3).

3. The food biopreservative composition of claim 1, wherein the consortium of lactic acid bacteria further comprises at least one probiotic lactic acid bacterium, and wherein:
(i) the at least one homofermentative lactic acid bacterium comprises at least one homofermentative lactic acid bacterium that produces proteases; and the at least one homofermentative lactic acid bacterium that produces proteases, the at least one heterofermentative lactic acid bacterium and the at least one probiotic lactic acid bacterium are in a ratio (1:1:1);
(ii) the at least one homofermentative lactic acid bacterium comprises at least one homofermentative lactic acid bacterium that produces proteases; and the at least one homofermentative lactic acid bacterium that produces proteases, the at least one heterofermentative lactic acid bacterium and at least one probiotic lactic acid bacterium are in a ratio (1:2:1); or
(iii) the at least one homofermentative lactic acid bacterium comprises at least one homofermentative lactic acid bacterium that produces proteases; and the at least one homofermentative lactic acid bacterium that produces proteases, the at least one heterofermentative lactic acid bacterium and the at least one probiotic lactic acid bacterium are in a ratio (1:3:1).

4. The food biopreservative composition of claim 3, wherein the at least one probiotic lactic acid bacterium is a homofermentative lactic acid bacterium; or wherein the probiotic lactic acid bacterium is *Bifidobacterium, Pediococcus, Lactobacillus reuteri, Lactobacillus johnsonii, Lactobacillus sake, Carnobacterium fish, Leuconostoc mesenteroides, Lactobacillus curvatus* or any combination of at least two thereof, or is in particular *Bifidobacterium, Lactobacillus reuteri, Lactobacillus johnsonii, Lactobacillus sake, Carnobacterium fish, Leuconostoc mesenteroides, Lactobacillus curvatus* or any combination of at least two thereof.

5. The food biopreservative composition of any one of claims 1 to 4, wherein (a) the homofermentative lactic acid bacterium is *Lactobacillus delbrueckii* subsp. *bulgaricus, Lactobacillus delbrueckii* subsp. *lactis, Lactobacillus delbrueckii subsp. leichmannii, Lactobacillus acidophilus, Lactobacillus amylophilus, Lactobacillus amylovorus*, *Lactobacillus animalis, Lactobacillus crispatus, Lactobacillus farciminis*, *Lactobacillus gasseri, Lactobacillus jensenii, Lactobacillus ruminis, Lactobacillus salivarius, Lactobacillus sharpeae, Lactobacillus vitulinus, Lactobacillus yamanashiensis* or any combination of at least two thereof; and (b) the heterofermentative bacterium is *Streptococcus thermophilus, Leuconostoc, Pediococcus, Lactobacillus plantarum*, *Lactobacillus rhamnosus, Lactobacillus helveticus* or any combination of at least two thereof; and/or (c) the consortium of lactic acid bacteria comprises *Lactobacillus delbrueckii subsp. bulgaricus* and *Streptococcus thermophilus,* and in particular the consortium of lactic acid bacteria further comprises *Bifidobacterium*; or the consortium of lactic acid bacteria consists of *Lactobacillus delbrueckii* subsp. *bulgaricus* and *Streptococcus thermophilus,* wherein most particularly the consortium of lactic acid bacteria consists of *Lactobacillus delbrueckii subsp. bulgaricus* and *Streptococcus thermophilus* in a ratio of 1:1.

6. The food biopreservative composition of any one of claims 1 to 5, wherein the concentration of each bacterial species of the consortium of lactic acid bacteria in the composition is between about 1x10⁴ and about 1x10⁸ CFU/ml; and/or wherein the total concentration of bacteria of the consortium of lactic acid bacteria in the composition is between about 2x10⁴ and about 5x10⁸ CFU/ml or between about 3.9x10⁶ and about 9.9 x10⁶ CFU/ml or is about 9.9 x10⁶ CFU/ml.

7. The food biopreservative composition of any one of claims 1 to 6, wherein the activating agent comprises starch, in particular wherein the starch
(i) is potato starch, yam starch, cassava starch, rice starch, corn starch, wheat starch, plantain starch or any combination of at least two thereof, and in particular is cassava starch, yam starch, or a combination thereof; and/or
(ii) (a) has an amylose content higher or equal to 10% (w/w); (b) has an amylopectin content lower or equal to 90% (w/w); or (c) has a ratio of amylose:amylopectin between about 17:83 and about 24:76 (w/w).

8. The food biopreservative composition of claim 7, wherein the starch is (a) cassava starch and wherein the concentration of the cassava starch in the composition is between about 0.05% (w/v) and about 1% (w/v), in particular wherein the concentration of the cassava starch in the composition is between about 0.1% (w/v) and about 0.3% (w/v); or
(b) yam starch and wherein the concentration of the yam starch in the composition is between about 0.1% (w/v) and about 2% (w/v), in particular wherein the concentration of the yam starch in the composition is about 0.3% (w/v).

9. The food biopreservative composition of any one of claims 1 to 6, wherein the activating agent is an extract from plantain peels and wherein the concentration of the extract in the composition is between about 0.1% (w/v) and about 20% (w/v), and in particular about 15% (w/v).

10. The food biopreservative composition any one of claims 1 to 6, wherein the activating agent comprises (i) a carbohydrate having from 3 to 5 carbon atoms, in particular wherein the carbohydrate is Xylitol, D-Ribose, D-Arabinose, D-lyxose, D-Xylose, D-Threose, D-Erytrose, D-Ribulose, D-Xylulose, D-Erythrulose or any combination of at least two thereof; or (ii) a fructooligosaccharide.

11. The food biopreservative composition 10, wherein the concentration of carbohydrate in the composition is between about 0.1% (w/v) and about 2% (w/v), in particular wherein the carbohydrate is xylitol and the concentration of xylitol is about 1.5% (w/v).

12. The food biopreservative composition of any one of claims 1 to 11, wherein the composition is in liquid form or in lyophilized form, and in particular further comprising a vehicle.

13. A kit for the preparation of a food biopreservative composition comprising (i) the consortium of lactic acid bacteria defined in any one of claims 1 to 12; and (ii) the activating agent defined in any one of claims 1 to 12,
in particular wherein the consortium of lactic acid bacteria is provided in lyophilized form.

14. Use of the composition defined in any one of claims 1 to 12, or of the kit defined in claim 13 (i) for increasing the shelf-life of food or for treating an inanimate surface which comes into contact with food; or (ii) for inhibiting the growth of a pathogenic microorganism in food or on an inanimate surface which comes into contact with food, wherein the pathogenic microorganism is in particular *E. coli, Salmonella spp, Listeria spp, Staphylococcus aureus, Clostridium, Aspergillus niger, Aspergillus flavus* or any combination of at least two thereof.

15. The composition of any one of claims 1 to 12, the kit of claim 13, or the use of claim 14, wherein the food is (a) fresh food, in particular raw meat, fresh vegetable or fruit; (b) processed and/or cooked food; or (iii) dry or wet food for pet.

## Patentansprüche

1. Biokonservierungszusammensetzung für Lebensmittel, umfassend (i) ein Konsortium von Milchsäurebakterien, umfassend mindestens ein homofermentatives Milchsäurebakterium und mindestens ein heterofermentatives Milchsäurebakterium; und (ii) ein Aktivierungsmittel, wobei das Aktivierungsmittel Stärke, ein Kohlenhydrat mit 3 bis 5 Kohlenstoffatomen ist oder umfasst ein Fructooligosaccharid und entweder in fester Form, in Lösung oder Dispersion in einer Konzentration zwischen 0,001 % und 20,0 % (Gew./Gew.) in der Zusammensetzung vorliegt, und wobei (a) das homofermentative Milchsäurebakterium *Lactobacillus delbrueckii* subsp. *bulgaricus, Lactobacillus delbrueckii* subsp. *lactis, Lactobacillus delbrueckii* subsp. *leichmannii, Lactobacillus acidophilus, Lactobacillus amylophilus, Lactobacillus amylovorus, Lactobacillus animalis, Lactobacillus crispatus, Lactobacillus farciminis, Lactobacillus gasseri, Lactobacillus jensenii, Lactobacillus ruminis, Lactobacillus salivarius, Lactobacillus sharpeae, Lactobacillus vitulinus, Lactobacillus yamanashiensis* oder eine beliebige Kombination von mindestens zwei davon ist; und/oder (b) das heterofermentative Bakterium *Streptococcus thermophilus, Leuconostoc, Pediococcus, Lactobacillus plantarum, Lactobacillus rhamnosus, Lactobacillus helveticus* oder eine beliebige Kombination von mindestens zwei davon ist.

2. Biokonservierungszusammensetzung für Lebensmittel nach Anspruch 1, wobei:
(i) das mindestens eine homofermentative Milchsäurebakterium Proteasen produziert; und das mindestens eine homofermentative Milchsäurebakterium und das mindestens eine heterofermentative Milchsäurebakterium in einem Verhältnis von (1:1) vorhanden sind;
(ii) das mindestens eine homofermentative Milchsäurebakterium Proteasen produziert; und das mindestens eine homofermentative Milchsäurebakterium und das mindestens eine heterofermentative Milchsäurebakterium in einem Verhältnis von (2:1) vorhanden sind;
(iii) das mindestens eine homofermentative Milchsäurebakterium Proteasen produziert; und das mindestens eine homofermentative Milchsäurebakterium und das mindestens eine heterofermentative Milchsäurebakterium in einem Verhältnis von (1:2) vorhanden sind;
(iv) das mindestens eine homofermentative Milchsäurebakterium ein probiotisches Milchsäurebakterium ist; und das mindestens eine homofermentative Milchsäurebakterium und das mindestens eine heterofermentative Milchsäurebakterium in einem Verhältnis von (1:3) vorhanden sind.

3. Biokonservierungszusammensetzung für Lebensmittel nach Anspruch 1, wobei das Konsortium von Milchsäurebakterien ferner mindestens ein probiotisches Milchsäurebakterium umfasst, und wobei:
(i) das mindestens eine homofermentative Milchsäurebakterium mindestens ein homofermentatives Milchsäurebakterium umfasst, das Proteasen produziert; und das mindestens eine homofermentative Milchsäurebakterium, das Proteasen produziert, das mindestens eine heterofermentative Milchsäurebakterium und das mindestens eine probiotische Milchsäurebakterium in einem Verhältnis (1:1:1) vorhanden sind;
(ii) das mindestens eine homofermentative Milchsäurebakterium mindestens ein homofermentatives Milchsäurebakterium umfasst, das Proteasen produziert; und das mindestens eine homofermentative Milchsäurebakterium, das Proteasen produziert, das mindestens eine heterofermentative Milchsäurebakterium und mindestens ein probiotisches Milchsäurebakterium in einem Verhältnis von (1:2:1) vorhanden sind; oder
(iii) das mindestens eine homofermentative Milchsäurebakterium mindestens ein homofermentatives Milchsäurebakterium umfasst, das Proteasen produziert; und das mindestens eine homofermentative Milchsäurebakterium, das Proteasen produziert, das mindestens eine heterofermentative Milchsäurebakterium und das mindestens eine probiotische Milchsäurebakterium in einem Verhältnis von (1:3:1) vorhanden sind.

4. Biokonservierungszusammensetzung für Lebensmittel nach Anspruch 3, wobei das mindestens eine probiotische Milchsäurebakterium ein homofermentatives Milchsäurebakterium ist; oder wobei das probiotische Milchsäurebakterium *Bifidobacterium, Pediococcus, Lactobacillus reuteri, Lactobacillus johnsonii, Lactobacillus sake, Carnobacterium fish, Leuconostoc mesenteroides, Lactobacillus curvatus* oder eine beliebige Kombination von mindestens zwei davon ist, oder insbesondere *Bifidobacterium, Lactobacillus reuteri, Lactobacillus johnsonii, Lactobacillus sake, Carnobacterium fish, Leuconostoc mesenteroides, Lactobacillus curvatus* oder eine beliebige Kombination von mindestens zwei davon ist.

5. Biokonservierungszusammensetzung für Lebensmittel nach einem der Ansprüche 1 bis 4, wobei (a) das homofermentative Milchsäurebakterium *Lactobacillus delbrueckii* subsp. *bulgaricus, Lactobacillus delbrueckii* subsp. *lactis, Lactobacillus delbrueckii* subsp. *leichmannii, Lactobacillus acidophilus, Lactobacillus amylophilus, Lactobacillus amylovorus, Lactobacillus animalis, Lactobacillus crispatus, Lactobacillus farciminis*, *Lactobacillus gasseri, Lactobacillus jensenii, Lactobacillus ruminis, Lactobacillus salivarius, Lactobacillus sharpeae, Lactobacillus vitulinus, Lactobacillus yamanashiensis* oder eine beliebige Kombination von mindestens zwei davon ist; und (b) das heterofermentative Bakterium *Streptococcus thermophilus, Leuconostoc, Pediococcus, Lactobacillus plantarum, Lactobacillus rhamnosus, Lactobacillus helveticus* oder eine beliebige Kombination von mindestens zwei davon ist; und/oder (c) das Konsortium von Milchsäurebakterien *Lactobacillus delbrueckii* subsp. *bulgaricus* und *Streptococcus thermophilus* umfasst, und das Konsortium von Milchsäurebakterien ferner insbesondere *Bifidobacterium* umfasst; oder das Konsortium von Milchsäurebakterien aus *Lactobacillus delbrueckii* subsp. *bulgaricus* und *Streptococcus thermophilus* besteht, wobei das Konsortium aus Milchsäurebakterien am meisten bevorzugt aus *Lactobacillus delbrueckii* subsp. *bulgaricus* und *Streptococcus thermophilus* in einem Verhältnis von 1:1 besteht.

6. Biokonservierungszusammensetzung für Lebensmittel nach einem der Ansprüche 1 bis 5, wobei die Konzentration jeder Bakterienspezies des Konsortiums von Milchsäurebakterien in der Zusammensetzung zwischen etwa 1x10⁴ und etwa 1x10⁸ CFU/ml liegt; und/oder wobei die Gesamtkonzentration von Bakterien des Konsortiums von Milchsäurebakterien in der Zusammensetzung zwischen etwa 2x10⁴ und etwa 5x10⁸ CFU/ml oder zwischen etwa 3,9x10⁶ und etwa 9,9x10⁶ CFU/ml liegt oder etwa 9,9x10⁶ CFU/ml beträgt.

7. Biokonservierungszusammensetzung für Lebensmittel nach einem der Ansprüche 1 bis 6, wobei das Aktivierungsmittel Stärke umfasst, wobei die Stärke insbesondere
(i) Kartoffelstärke, Yamswurzelstärke, Maniokstärke, Reisstärke, Maisstärke, Weizenstärke, Kochbananenstärke oder eine beliebige Kombination von mindestens zwei davon ist, insbesondere Maniokstärke, Yamswurzelstärke oder eine beliebige Kombination davon ist; und/oder
(ii) (a) einen Amylosegehalt von größer oder gleich 10 % (Gew./Gew.) aufweist; (a) einen Amylospektingehalt von weniger oder gleich 90 % (Gew./Gew.) aufweist; oder (c) ein Verhältnis von Amylose:Amylopektin zwischen etwa 17:83 und etwa 24:76 (Gew./Gew.) aufweist.

8. Biokonservierungszusammensetzung für Lebensmittel nach Anspruch 7, wobei die Stärke (a) Maniokstärke ist und wobei die Konzentration der Maniokstärke in der Zusammensetzung zwischen etwa 0,05 % (Gew./Vol.) und etwa 1 % (Gew./Vol.) liegt, wobei die Konzentration der Maniokstärke in der Zusammensetzung insbesondere zwischen etwa 0,1 % (Gew./Vol.) und etwa 0,3 % (Gew./Vol.) liegt; oder
(b) Yamswurzelstärke und wobei die Konzentration der Yamswurzelstärke in der Zusammensetzung zwischen etwa 0,1 % (Gew./Vol.) und etwa 2 % (Gew./Vol.) liegt, wobei die Konzentration der Yamswurzelstärke in der Zusammensetzung insbesondere etwa 0,3 % (Gew./Vol.) beträgt.

9. Biokonservierungszusammensetzung für Lebensmittel nach einem der Ansprüche 1 bis 6, wobei das Aktivierungsmittel ein Extrakt aus Kochbananenschalen ist und wobei die Konzentration des Extrakts in der Zusammensetzung zwischen etwa 0,1 % (Gew./Vol.) und etwa 20 % (Gew./Vol.) liegt und insbesondere etwa 15 % (Gew./Vol.) beträgt.

10. Biokonservierungszusammensetzung für Lebensmittel nach einem der Ansprüche 1 bis 6, wobei das Aktivierungsmittel (i) ein Kohlenhydrat mit 3 bis 5 Kohlenstoffatomen umfasst, wobei das Kohlenhydrat insbesondere Xylitol, D-Ribose, D-Arabinose, D-Lyxose, D-Xylose, D-Threose, D-Erytrose, D-Ribulose, D-Xylulose, D-Erythrulose oder eine beliebige Kombination von mindestens zwei davon ist; oder (ii) ein Fructooligosaccharid.

11. Biokonservierungszusammensetzung 10, wobei die Kohlenhydratkonzentration in der Zusammensetzung zwischen etwa 0,1 % (Gew./Vol.) und etwa 2 % (Gew./Vol.) liegt, wobei das Kohlenhydrat insbesondere Xylitol ist und die Konzentration von Xylitol etwa 1,5 % (Gew./Vol.) beträgt.

12. Biokonservierungszusammensetzung für Lebensmittel nach einem der Ansprüche 1 bis 11, wobei die Zusammensetzung in flüssiger Form oder in lyophilisierter Form vorliegt, und insbesondere ferner ein Vehikel umfasst.

13. Kit für die Herstellung einer Biokonservierungszusammensetzung für Lebensmittel, umfassend (i) das Konsortium von Milchsäurebakterien nach einem der Ansprüche 1 bis 12; und (ii) das Aktivierungsmittel nach einem der Ansprüche 1 bis 12, wobei das Konsortium von Milchsäurebakterien insbesondere in lyophilisierter Form bereitgestellt wird.

14. Verwendung der Zusammensetzung nach einem der Ansprüche 1 bis 12 oder des Kits nach Anspruch 13 (i) zur Verlängerung der Haltbarkeit von Lebensmitteln oder zur Behandlung einer unbelebten Oberfläche, die mit Lebensmitteln in Berührung kommt; oder (ii) zur Hemmung des Wachstums eines pathogenen Mikroorganismus in Lebensmitteln oder auf einer unbelebten Oberfläche, die mit Lebensmitteln in Berührung kommt, wobei der pathogene Mikroorganismus insbesondere *E. coli, Salmonella spp, Listeria spp,* Staphylococcus *aureus, Clostridium, Aspergillus niger, Aspergillus flavus* oder eine beliebige Kombination von mindestens zwei davon ist.

15. Zusammensetzung nach einem der Ansprüche 1 bis 12, Kit nach Anspruch 13 oder Verwendung nach Anspruch 14, wobei das Lebensmittel (a) ein frisches Lebensmittel, insbesondere rohes Fleisch, frisches Gemüse oder Obst; b) ein verarbeitetes und/oder ein zubereitetet Lebensmittel ist; oder (iii) Trocken- oder Nassfutter für Haustiere.

## Revendications

1. Composition de bio-conservateur alimentaire comprenant (i) un consortium de bactéries lactiques comprenant au moins une bactérie lactique homofermentaire et au moins une bactérie lactique hétérofermentaire ; et (ii) un agent d'activation, dans laquelle l'agent d'activation est de l'amidon, un hydrate de carbone ayant de 3 à 5 atomes de carbone ou comprend un fructooligosaccharide et est soit sous forme solide, soit en solution ou en dispersion à une concentration comprise entre 0.001% et 20.0% (p/p) dans la composition, et dans laquelle (a) la bactérie lactique homofermentaire est *Lactobacillus delbrueckii* subsp. *bulgaricus, Lactobacillus delbrueckii* subsp. *lactis, Lactobacillus delbrueckii subsp. leichmannii, Lactobacillus acidophilus, Lactobacillus amylophilus, Lactobacillus amylovorus*, *Lactobacillus animalis, Lactobacillus crispatus, Lactobacillus farciminis, Lactobacillus gasseri, Lactobacillus jensenii, Lactobacillus ruminis, Lactobacillus salivarius, Lactobacillus sharpeae, Lactobacillus vitulinus, Lactobacillus yamanashiensis* ou toute combinaison d'au moins deux de ces bactéries ; et/ou (b) la bactérie hétérofermentaire est *Streptococcus thermophilus, Leuconostoc, Pediococcus, Lactobacillus plantarum*, *Lactobacillus rhamnosus, Lactobacillus helveticus* ou toute combinaison d'au moins deux de ces bactéries.

2. Composition de bio-conservateur alimentaire selon la revendication 1, dans laquelle :
(i) la au moins une bactérie lactique homofermentaire produit des protéases ; et la au moins une bactérie lactique homofermentaire et la au moins une bactérie lactique hétérofermentaire sont dans un rapport (1:1) ;
(ii) la au moins une bactérie lactique homofermentaire produit des protéases ; et la au moins une bactérie lactique homofermentaire et la au moins une bactérie lactique hétérofermentaire sont dans un rapport (2:1) ;
(iii) la au moins une bactérie lactique homofermentaire produit des protéases ; et la au moins une bactérie lactique homofermentaire et la au moins une bactérie lactique hétérofermentaire sont dans un rapport (1:2) ;
(iv) la au moins une bactérie lactique homofermentaire est une bactérie lactique probiotique ; et la au moins une bactérie lactique homofermentaire et la au moins une bactérie lactique hétérofermentaire sont dans un rapport (1:3).

3. Composition de bio-conservateur alimentaire selon la revendication 1, dans lequel le consortium de bactéries lactiques comprend en outre au moins une bactérie lactique probiotique, et dans lequel :
(i) la au moins une bactérie lactique homofermentaire comprend au moins une bactérie lactique homofermentaire qui produit des protéases ; et la au moins une bactérie lactique homofermentaire qui produit des protéases, la au moins une bactérie lactique hétérofermentaire et la au moins une bactérie lactique probiotique sont dans un rapport (1:1:1) ;
(ii) la au moins une bactérie lactique homofermentaire comprend au moins une bactérie lactique homofermentaire qui produit des protéases ; et la au moins une bactérie lactique homofermentaire qui produit des protéases, la au moins une bactérie lactique hétérofermentaire et la au moins une bactérie lactique probiotique sont dans un rapport (1:2:1) ; ou
(iii) la au moins une bactérie lactique homofermentaire comprend au moins une bactérie lactique homofermentaire qui produit des protéases; et la au moins une bactérie lactique homofermentaire qui produit des protéases, la au moins une bactérie lactique hétérofermentaire et la au moins une bactérie lactique probiotique sont dans un rapport (1:3:1).

4. Composition de bio-conservateur alimentaire selon la revendication 3, dans laquelle la au moins une bactérie lactique probiotique est une bactérie lactique homofermentaire ; ou dans laquelle la bactérie lactique probiotique est *Bifidobacterium, Pediococcus, Lactobacillus reuteri, Lactobacillus johnsonii, Lactobacillus sake, Carnobacterium fish, Leuconostoc mesenteroides, Lactobacillus curvatus* ou toute combinaison d'au moins deux de ces bactéries, ou est notamment *Bifidobacterium, Lactobacillus reuteri, Lactobacillus johnsonii, Lactobacillus sake, Carnobacterium fish*, *Leuconostoc mesenteroides, Lactobacillus curvatus* ou toute combinaison d'au moins deux de ces bactéries.

5. Composition de bio-conservateur alimentaire selon l'une quelconque des revendications 1 à 4, dans laquelle (a) la bactérie lactique homofermentaire est *Lactobacillus delbrueckii* subsp. *bulgaricus, Lactobacillus delbrueckii* subsp. *lactis, Lactobacillus delbrueckii subsp. leichmannii, Lactobacillus acidophilus, Lactobacillus amylophilus*, *Lactobacillus amylovorus*, *Lactobacillus animalis, Lactobacillus crispatus, Lactobacillus farciminis, Lactobacillus gasseri, Lactobacillus jensenii, Lactobacillus ruminis, Lactobacillus salivarius, Lactobacillus sharpeae, Lactobacillus vitulinus, Lactobacillus yamanashiensis* ou toute combinaison d'au moins deux de ces bactéries ; et (b) la bactérie hétérofermentaire est *Streptococcus thermophilus, Leuconostoc, Pediococcus, Lactobacillus plantarum*, *Lactobacillus rhamnosus, Lactobacillus helveticus* ou toute combinaison d'au moins deux ces bactéries ; et/ou (c) le consortium de bactéries lactiques comprend *Lactobacillus delbrueckii subsp. bulgaricus* et *Streptococcus thermophilus,* et en particulier le consortium de bactéries lactiques comprend en outre *Bifidobacterium ;* ou le consortium de bactéries lactiques est constitué de *Lactobacillus delbrueckii* subsp. *bulgaricus* et *Streptococcus thermophilus,* dans laquelle plus particulièrement le consortium de bactéries lactiques est constitué de *Lactobacillus delbrueckii subsp. bulgaricus* et *Streptococcus thermophilus* dans un rapport 1:1.

6. Composition de bio-conservateur alimentaire selon l'une quelconque des revendications 1 à 5, dans laquelle la concentration de chaque espèce bactérienne du consortium de bactéries lactiques dans la composition est comprise entre environ 1x10⁴ et environ 1x10⁸ CFU/ml ; et/ou dans laquelle la concentration totale de bactéries du consortium de bactéries lactiques dans la composition est comprise entre environ 2x10⁴ et environ 5x10⁸ CFU/ml ou comprise entre environ 3.9x10⁶ et environ 9.9 x10⁶ CFU/ml ou est d'environ 9.9 x10⁶ CFU/ml.

7. Composition de bio-conservateur alimentaire selon l'une quelconque des revendications 1 à 6, dans laquelle l'agent d'activation comprend de l'amidon, en particulier dans laquelle l'amidon
(i) est la fécule de pomme de terre, la fécule d'igname, la fécule de manioc, la fécule de riz, la fécule de maïs, la fécule de blé, la fécule de plantain ou toute combinaison d'au moins deux d'entre elles, et notamment la fécule de manioc, la fécule d'igname ou une combinaison de celles-ci ; et/ou
(ii) (a) a une teneur en amylose supérieure ou égale à 10% (p/p) ; (b) a une teneur en amylopectine inférieure ou égale à 90% (p/p) ; ou (c) a un rapport amylose:amylopectine compris entre environ 17:83 et environ 24:76 (p/p).

8. Composition de bio-conservateur alimentaire selon la revendication 7, dans laquelle l'amidon est (a) de l'amidon de manioc et dans laquelle la concentration de l'amidon de manioc dans la composition est comprise entre environ 0,05% (p/v) et environ 1% (p/v), en particulier dans laquelle la concentration de l'amidon de manioc dans la composition est comprise entre environ 0,1% (p/v) et environ 0,3% (p/v) ; ou
(b) de l'amidon d'igname et dans lequel la concentration de l'amidon d'igname dans la composition est comprise entre environ 0,1% (p/v) et environ 2% (p/v), en particulier dans lequel la concentration de l'amidon d'igname dans la composition est d'environ 0,3% (p/v).

9. Composition de bio-conservateur alimentaire selon l'une quelconque des revendications 1 à 6, dans laquelle l'agent activateur est un extrait de pelures de plantain et dans laquelle la concentration de l'extrait dans la composition est comprise entre environ 0,1% (p/v) et environ 20% (p/v), et en particulier environ 15% (p/v).

10. Composition de bio-conservateur alimentaire selon l'une quelconque des revendications 1 à 6, dans laquelle l'agent d'activation comprend (i) un glucide ayant de 3 à 5 atomes de carbone, en particulier dans lequel le glucide est le Xylitol, le D-Ribose, le D-Arabinose, le D-lyxose, le D-Xylose, le D-Threose, le D-Erytrose, le D-Ribulose, le D-Xylulose, le D-Erythrulose ou toute combinaison d'au moins deux de ceux-ci ; ou (ii) un fructooligosaccharide.

11. Composition de bio-conservateur alimentaire selon la revendication 10, dans laquelle la concentration d'hydrate de carbone dans la composition est comprise entre environ 0,1% (p/v) et environ 2% (p/v), en particulier dans laquelle l'hydrate de carbone est le xylitol et la concentration de xylitol est d'environ 1,5% (p/v).

12. Composition de bio-conservateur alimentaire selon l'une quelconque des revendications 1 à 11, dans laquelle la composition est sous forme liquide ou sous forme lyophilisée, et comprenant en outre un véhicule.

13. Kit pour la préparation d'une composition de bio-conservateur alimentaire comprenant (i) le consortium de bactéries lactiques défini dans l'une quelconque des revendications 1 à 12 ; et (ii) l'agent d'activation défini dans l'une quelconque des revendications 1 à 12,
en particulier dans lequel le consortium de bactéries lactiques est fourni sous forme lyophilisée.

14. Utilisation de la composition définie dans l'une quelconque des revendications 1 à 12, ou du kit défini dans la revendication 13 (i) pour augmenter la durée de conservation des denrées alimentaires ou pour traiter une surface inanimée qui entre en contact avec des denrées alimentaires ; ou (ii) pour inhiber la croissance d'un micro-organisme pathogène dans les denrées alimentaires ou sur une surface inanimée qui entre en contact avec des denrées alimentaires, dans laquelle le micro-organisme pathogène est en particulier *E*. *coli, Salmonella spp, Listeria spp, Staphylococcus aureus, Clostridium, Aspergillus niger, Aspergillus flavus* ou toute combinaison d'au moins deux de ces micro-organismes.

15. Composition selon l'une quelconque des revendications 1 à 12, kit selon la revendication 13 ou utilisation selon la revendication 14, dans laquelle la denrée alimentaire est (a) une denrée alimentaire fraîche, en particulier de la viande crue, des légumes ou des fruits frais ; (b) une denrée alimentaire transformée et/ou cuite ; ou (iii) une denrée alimentaire sèche ou humide pour animaux de compagnie.
